# EUROPEAN PATENT SPECIFICATION

(11) **EP 2 513 135 B1**
(45) Date of publication and mention of the grant of the patent: **22.04.2020**
(21) Application number: 10798291.0
(22) Date of filing: 15.12.2010
(51) Int. Cl.: C07K 1/22, C12N 9/64

(54) **METHOD OF PURIFYING POLYPEPTIDES**
Verfahren zur Reinigung von Polypeptiden
Procédé de purification de polypeptides

(30) Priority: 18.12.2009 EP 09015707
(43) Date of publication of application: 24.10.2012
(60) Divisional application: 20162007.7
(73) Proprietor: CSL Limited, Parkville VIC 3052 (AU)
(72) Inventor: CHARLTON, Adam, Collinswood, South Australia 5081 (AU); NAPOLI, Mark, Hoppers Crossing Victoria 3029 (AU); SMRDELJ, Paul, Greenvale Victoria 3059 (AU); STOWERS, Anthony, Preston Victoria 3072 (AU); PIRZAS, Vicky, 35041 Marburg (DE); SCHRÖDER, Magnus, Doncaster East, Victoria 3109 (AU); WALKER, Ian, Victoria, 3145 (AU)
(74) Representative: Hauser, Hans-Peter
(86) International application number: PCT/EP2010/069713
(87) International publication number: WO 2011/073235

(56) References cited:
- EP-A1- 0 829 537
- WO-A1-00/55203
- WO-A1-01/37861
- WO-A2-2004/006962
- GB-A- 2 440 273
- TAUC P ET AL: "Ion-exchange chromatography of proteins: modulation of selectivity by addition of organic solvents to mobile phase - Application to single-step purification of a proteinase inhibitor from corn and study of the mechanism of selectivity modulation", JOURNAL OF CHROMATOGRAPHY, ELSEVIER SCIENCE PUBLISHERS B.V, NL LNKD- DOI:10.1016/S0021-9673(98)00688-8, vol. 825, no. 1, 30 October 1998 (1998-10-30), pages 17-27, XP004141938, ISSN: 0021-9673
- GAGNON P ET AL: "Method for obtaining unique selectivities in ion-exchange chromatography by addition of organic polymers to the mobile phase", JOURNAL OF CHROMATOGRAPHY, ELSEVIER SCIENCE PUBLISHERS B.V, NL LNKD- DOI:10.1016/0021-9673(96)00125-2, vol. 743, no. 1, 30 August 1996 (1996-08-30), pages 51-55, XP004020270, ISSN: 0021-9673

## Description

### Field of the Invention

The present invention relates to improved processes for purifying polypeptides of interest by increasing the amount of a polypeptide of interest bound to a cation-exchange matrix relative to the amount of one or more impurities bound to the ion-exchange matrix. This effect is achieved by adding a chemical compound in the process which by also binding to the ion-exchange matrix reduces the binding of impurities more than the binding of the polypeptide of interest.

The present invention is defined by the claims. Any subject matter falling outside the scope of the claims is provided for information only.

### Background to the Invention

The large-scale, economic purification of polypeptides is increasingly an important problem for the biotechnology industry. Polypeptides are produced by cell culture, using either mammalian or bacterial cell lines engineered to produce the polypeptide of interest by insertion of a recombinant plasmid containing the gene for that polypeptide. Since the cell lines used are living organisms, they are often fed with a complex growth medium, containing sugars, amino acids, and growth factors, in some cases also supplied from preparations of animal serum. Separation of the desired polypeptide of interest from the mixture of compounds fed to the cells and from the by-products of the cells themselves to a purity sufficient for use as a human therapeutic poses a formidable challenge.

Recombinant therapeutic polypeptides are commonly produced in several mammalian host cell lines including Chinese Hamster Ovary (CHO) cells, Baby Hamster Kidney (BHK) cells, Human Embryonic Kidney (HEK) cells, murine myeloma NSO cells, yeast cells and bacterial cells such as E.coli and insect cells. Each cell line has advantages and disadvantages in terms of productivity and the characteristics of the polypeptides produced by the cells. Choices of commercial production cell lines often balance the need for high productivity with the ability to deliver the product quality attributes required of a given product. Advances in fermentation and cell culture techniques have greatly increased the concentrations of target polypeptides in culture fluid. This increase in upstream efficiency has led to a bottleneck in downstream processing at the cell-harvest stage. Cell harvesting, or clarification of the harvested cell culture fluid, is an important process in nearly all downstream purifications of biotech-based products.

Once the polypeptide is expressed at the desired levels, the polypeptide is removed from the host cell and harvested. Suspended particulates, such as cells, cell fragments, lipids and other insoluble matter are typically removed from the polypeptide-containing fluid by filtration or centrifugation, resulting in a clarified fluid containing the polypeptide of interest in solution as well as other soluble impurities. Procedures for purification of polypeptides from cell debris initially depend on the site of expression of the polypeptide. Some polypeptides are caused to be secreted directly from the cell into the surrounding growth media; others are made intracellularly. For the latter polypeptides, the first step of a purification process involves lysis of the cell, which can be done by a variety of methods, including mechanical shear, osmotic shock, or enzymatic treatments. Such disruption releases the entire contents of the cell into the homogenate, and in addition produces subcellular fragments that are difficult to remove due to their small size. These are generally removed by centrifugation or by filtration. The same problem arises, although on a smaller scale, with directly secreted polypeptides due to the natural death of cells and release of intracellular host cell polypeptides in the course of the polypeptide production run.

Impurities including host cell polypeptides, product variants, host cell DNA, small molecules, process related contaminants, endotoxins, prions and viral particles must be removed The purification techniques used must be scaleable, efficient, cost- effective, reliable, and meet the rigorous purity requirements of the final product. Current purification techniques typically involve multiple chromatographic separations. A typical process might include all or at least some of the following steps: precipitation, dialysis, electrophoresis, ultrafiltration, affinity chromatography, cation exchange chromatography, anion exchange chromatography and hydrophobic interaction chromatography. Conventional column chromatography steps are effective and reliable, but generally have low product throughput.

Once a solution containing the polypeptide of interest is obtained, its separation from the other polypeptides produced by the cell is usually attempted using a combination of different chromatography techniques. In some cases, the desired polypeptide is separated from impurities whereby the impurities specifically adhere to the column, and the polypeptide of interest does not, that is, the polypeptide of interest is present in the "flow- through" ("negative-mode" chromatography).

Chromatography techniques exploit the chemical and physical properties of polypeptides to achieve a high degree of purification. These chemical and physical properties typically include size, isoelectric point, charge distribution, hydrophobic sites and affinity for ligands (Janson, J. C. and L. Ryden (eds.). (1989) Polypeptide Purification: Principles, High Resolution Methods and Applications. VCH Publishers, Inc., New York). The various separation modes of chromatography include: ion-exchange, chromatofocusing, gel filtration (size exclusion), hydrophobic interaction, reverse phase, and affinity chromatography. Ion-exchange chromatography (IEX), including anion-exchange chromatography (AEX) and cation-exchange chromatography (CEX) separates analytes (e.g. polypeptides) by differences of their net surface charges. IEX is a primary tool for the separation of expressed polypeptides from cellular debris and other impurities. Today, IEX is one of the most frequently used techniques for purification of polypeptides, peptides, nucleic acids and other charged biomolecules, offering high resolution and group separations with high loading capacity. The technique is capable of separating molecular species that have only minor differences in their charge properties, for example two polypeptides differing by one charged amino acid. These features make IEX well suited for capture, intermediate purification or polishing steps in a purification protocol and the technique is used from microscale purification and analysis through to purification of kilograms of product.

IEX, named for the exchangeable counterion, is a procedure applicable to purification of ionizable molecules. Ionized molecules are separated on the basis of the non-specific electrostatic interaction of their charged groups with oppositely charged molecules attached to the solid phase support matrix, thereby retarding those ionized molecules that interact more strongly with the solid phase. The net charge of each type of ionized molecule, and its affinity for the matrix, varies according to the number of charged groups, the charge of each group, and the nature of the molecules competing for interaction with the charged solid phase matrix. These differences result in resolution of various molecule types by IEX. In a typical polypeptide purification using IEX, a mixture of many polypeptides derived from a host cell, such as in mammalian cell culture, is applied to an ion-exchange column. After non-binding molecules are washed away, conditions are adjusted, such as by changing pH, counter ion concentration and the like in a step-wise or in a gradient-mode, to release from the solid phase a non-specifically retained or retarded ionized polypeptide of interest and separating it from polypeptides having different charge characteristics. AEX involves competition of an anionic molecule of interest with negative ions for interaction with a positively charged molecule attached to the solid phase matrix at the pH and under the conditions of a particular separation process. By contrast, CEX involves competition of a cationic molecule of interest with positive ions for a negatively charged molecule attached to the solid phase matrix at the pH and under the conditions of a particular separation process. Mixed mode ion exchange chromatography involves the use of a combination of ion exchange chromatographic and hydrophobic interaction chromatography media in the same step. In particular, "mixed-mode" refers to a solid phase support matrix to which is covalently attached a mixture of hydrophobic interaction and either cation exchange or anion exchange moieties.

Vitamin K-dependent polypeptides are distinguished from other polypeptides by sharing a common structural feature in the amino terminal part of the molecule. The N-terminal part of these polypeptides, also referred to as the Gla-domain, is rich in the unusual amino acid gamma-carboxy glutamic acid which is synthesized from glutamate in a Vitamin K-dependent reaction catalysed by the enzyme gamma-glutamyl carboxylase. Because of the presence of about 2 to 12 Gla residues, the Gla-domain is characterised by being capable of binding divalent cations such as Ca²⁺. Upon binding of metal ions, these polypeptides undergo conformational changes which can be measured by several techniques such as circular dichroism and fluorescence emission. In the 1980's conformation specific pseudoaffinity chromatography was developed making use of the unique property of Gla containing polypeptides to undergo metal induced changes in conformation. Pseudoaffinity chromatography differs from the conventional affinity chromatography in that there is no immobilized affinity ligand involved and it is performed on a conventional chromatographic matrix (Yan S. B., J. Mol. Recog. 1996; 9, 211-218). The Gla polypeptide can be adsorbed to an anion exchange material by eliminating divalent metal ions. Subsequently, elution is performed by adding Ca²⁺ to the elution buffer.

In 1986, Bjoern and Thim reported purification of recombinant Factor VII on an anion exchange material taking advantage of Ca²⁺-binding property of Gla-domain of Factor VII (Bjoern S. and Thim L., Research Dislosure, 1986, 26960-26962.). Adsorption was achieved in a buffer without Ca²⁺and elution of Factor VII was possible using a Ca²⁺ containing buffer with low ionic strength and under mild conditions. Yan et al. have used the same principle for the purification of recombinant human Polypeptide C (Yan S. B. et al., Bio/technology. 1990; 8, 655-661).

Polypeptides with a GLA-domain comprise, but are not limited to, the following polypeptides: GAS-6, Polypeptide S, Factor II (Prothrombin), Thrombin, Factor X/Xa, Factor IX/IXa, Polypeptide C, Factor VII/VIIa, Polypeptide Z, Transmembrane gamma-carboxyglutamic acid polypeptide 1, Transmembrane gamma-carboxyglutamic acid polypeptide 2, Transmembrane gamma carboxyglutamic acid polypeptide 3, Transmembrane gamma-carboxyglutamic acid polypeptide 4, Matrix Gla polypeptide, and Osteocalcin.

In the art several attempts to reduce impurities from polypeptides of interest have been described.

WO 2009/082443 relates to a polymer such as a soluble polymer capable of irreversibly binding to insoluble particulates and a subset of soluble impurities and also capable of reversibly binding to one or more desired biomolecules in an unclarified biological material containing stream and the methods of using such a material to purify one or more desired biomolecules from such a stream without the need for prior clarification.

Only when precipitated out of solution, the polymer is capable of reversibly binding to one or more desired biomolecules within the stream (polypeptide, polypeptide, etc) in an unclarified cell broth. The precipitate can then be removed from the stream, such as by being filtered out from the remainder of the stream and the desired biomolecule is recovered such as by selective elution from the precipitate. The stream is then discarded removing with it the great majority of the impurities of the mixture such as cell culture media, anti foam materials, additives, and soluble components.

WO 2008/122089 discloses the preferential precipitation of contaminating polypeptides, including host polypeptides and cleaved fusion partners, leaving the recombinant polypeptide in solution for subsequent recovery. Thus a solution comprising both the peptide of interest and soluble contaminating polypeptides is subjected to conditions which result in preferential precipitation of the contaminating polypeptides (e.g. host cell polypeptides, cleaved polypeptide fusion partner and polypeptideaceous cleavage agents such as proteases). The precipitate can then be separated from the solution containing the peptide of interest which can then be recovered from that solution by suitable techniques such as freeze drying etc.

WO 2008/091740 provides methods relating to the isolation and purification of polypeptides derived from cell culture fluids by precipitation of a polypeptide with a polyelectrolyte, such as a polyanion polyelectrolyte or with a polycation polyelectrolyte. The precipitation step may be followed by cation exchange chromatography, anion exchange chromatography, and other precipitation steps.

WO 2008/031020 relates to methods for isolating a product from a loading fluid that contains a product, such as an antibody, and one or more impurities by passing the loading fluid through a medium that binds the product, followed by passing at least one wash solution containing arginine or an arginine derivative through the medium, and collecting the product using an elution solution.

WO 2007/117490 relates to a method for producing a host cell polypeptide-(HCP) reduced antibody preparation from a mixture comprising an antibody and at least one HCP, comprising an ion exchange separation step wherein the mixture is subjected to a first ion exchange material by applying more than 30 grams of antibody per liter of matrix.

WO 2007/108955 relates to the purification of polypeptides and polypeptides, in particular recombinant polypeptides such as monoclonal antibodies from a contaminated mixture containing contaminants such as host cell polypeptides and media components by using a two-step purification process that does not include an affinity chromatography step or an in-process buffer exchange step (e.g., TFF or HPTFF). Rather, only pH manipulations and/or dilutions are necessary from the first step to the second step.

WO 2007/071768 relates to a method for purifying a polypeptide from a composition, the method comprising loading a solution of said composition onto a reversed phase liquid chromatography column and eluting said polypeptide from the column with a solvent containing a buffer and a salt, wherein said salt does not have buffering capacity at the pH of the buffer used. The method further provides a gentle way of purifying polypeptides in industrial-scale, i.e. a method wherein a substantial amount of the loaded polypeptide survives the operating conditions and retains its bioactivity.

WO 2006/110277 relates to the purification of a molecule of interest from at least one contaminant, such as host cell polypeptides and other materials such as media components, by using a two-step purification process that does not include an affinity chromatography step or an in-process buffer exchange step (e.g., TFF or HPTFF). Rather, only pH manipulations are necessary from the first step to the second step. HCP CHOP levels were reduced to less than 20 ppm.

WO 2006/067230 relates to various methods for reducing or even eliminating the content of polypeptide contaminant(s) in compositions comprising a Vitamin K-dependent polypeptide of interest. In a preferred embodiment Protein S originating from the host cells is removed.

WO 2003/102132 relates to a combination of a non-affinity chromatographic purification process in combination with high-performance tangential-flow filtration (HPTFF) is capable of purifying a target polypeptide, such as an antibody or an antibody-like molecule, from a mixture containing host cell polypeptides such that host cell polypeptide impurities are present in the final purified target polypeptide in an amount less than 100 parts per million (ppm).

The use of chemical compounds in IEX which do have multiple charges opposite to the ion-exchange matrix of choice to reduce impurities like host cell protein, host cell nucleic acids, endotoxins, viruses while purifying a polypeptide of interest has to the best knowledge of the inventors not yet been described in the prior art.

AEX has been described as a means to reduce endotoxins from recombinant therapeutic proteins (Chen et al., 2009, Protein Expression and Purification, pp 76-81) but the use of positively charged chemical compounds to enhance the reduction of endotoxins is not mentioned.

EGTA has been shown to bind to an anion exchange resion when applied in a concentration of 2mM (Yingst et al, 1994, Biochimica et Biophysica Acta 1189, pp 113-118).

EDTA has been used in a concentration between 1mM and 10mM as a means to inhibit proteases (Charlton A, Methods in Molecular Biology, vol. 241 Affinity Chromatography: Methods and Protocols, Second Edition, Humana Press, pp 211-227) and in a concentration of 10mM to solubilize bacterial inclusion bodies (Ledung et al., 2009, J.Biotechnology, 141, pp 64-72) and to reduce aggregation of the polypeptide of interest.

In IEX EDTA was used at at a concentration of 1mM in the purification of thombospondin in an anion exchange Mono-Q column finding no change in the chromatography profiles, or at a 5 mM concentration without giving a reason for its use in Zhu (Zhu et al., 2009, Process Biochemistry 44 pp 875-879), Chen (Chen et al. 2009, J.Chromatorgr. A 1216, pp 4877-4886) suggested the use of 1 mM EDTA for refolding on an anion-exchange material bacterially expressed polypeptides of interest. Haganika (Haganika et al., 2001, J.Chromatography B, 751, pp 161-167) used 1 mM in elution buffers from an anion exchange matrix also without explaining the rational for doing so. Pittalis et al. (Pittalis et al., 1992, J.Chromatography, 573 pp 29-34.) used 1,25 mM EDTA concentrations in order equilibrate an anion exchange matrix. Häberlein (Häberlein, 1991, J. Chromatography, 587, pp 109-115) used 2 mM of EDTA in the purification of thioredoxin using the anion-exchange matrix Mono-Q. Basta (Basta et al., 1991, J.Immunological Methods, 142, pp39-44) used a buffer containing 6.5 mM EDTA for elution of a complement protein from a Mono Q column.

None of the above uses mentioned a positive effect on the performance of an ion-exchange based purification of a polypeptide of interest.

A paper from Nielsen et al (Nielsen et al., 1985, Veterinary Immunology and Immunopathology, 9, pp 349-359) mentions a beneficial effect of the addition of 1 mM EDTA improved separation between IgG1, IgG2, IgM and albumin on some matrices but not all. The authors suggested a role for EDTA preventing protein association, thereby improving column ligand binding.

So in summary to the best knowledge of the inventors of the present invention the highest levels of a multiply charged chemical compound in addition to the polypeptide of interest used so far in IEX were 6.5 mM EDTA in AEX.

### Summary of the Invention

The present invention is defined by the claims and relates to improved processes for purifying polypeptides of interest from complex mixtures by increasing the amount of a polypeptide of interest bound to a cation-exchange matrix relative to the amount of one or more impurities bound to the ion-exchange matrix. This effect is achieved by adding a chemical compound in the process which by also binding to the ion-exchange matrix reduces the binding of impurities more than the binding of the polypeptide of interest. The complex mixtures above may result from recombinant production schemes or be e.g. human or animal body fluids, preferably blood or plasma solutions.

One embodiment of the invention is the use of a chemical compound to reduce impurities in a polypeptide of interest in IEX wherein at the selected purification conditions the chemical compound binds to the ion-exchange matrix due to a charge that is opposite to the charge of the ion-exchange matrix thereby increasing the specificity of binding of a polypeptide of interest by reducing the amount of impurities bound to the ion-exchange matrix more than the amount of the polypeptide of interest bound to the ion-exchange matrix.

The chemical compound is positively charged and the ion-exchange matrix is a cation exchange matrix.

In yet another embodiment the invention relates to a purification process in which a chemical compound reduces impurities in a polypeptide of interest in IEX wherein at the selected purification conditions the chemical compound binds to the ion-exchange matrix due to a charge that is opposite to that of the ion-exchange matrix thereby increasing the specificity of binding of a polypeptide of interest by reducing the amount of impurities bound to the ion-exchange matrix more than the amount of the polypeptide of interest bound to the ion-exchange matrix and wherein the chemical compound is used at a concentration of at least 7mM.

### Detailed Description of the Invention

Reference will now be made in detail to certain embodiments of the invention. The invention will be described in conjunction with the enumerated embodiments.

Unless defined otherwise, all technical and scientific terms used herein have the same meaning as commonly understood by one of ordinary skill in the art (e.g. in cell biology, chemistry and molecular biology).

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Throughout this specification, reference to numerical values, unless stated otherwise, is to be taken as meaning "about" that numerical value. The term "about" is used to indicate that a value includes the inherent variation of error for the device and the method being employed to determine the value, or the variation that exists among the study subjects.

Temperature values are given for conditions of standard pressure (1atm).

The invention is based on the surprising finding that during IEX chemical compounds can be added to the equilibration fluid and/or to the sample fluid and/or to the wash fluid which compete with the binding of a polypeptide of interest and with the binding of impurities to the ion-exchange matrix. It was surprisingly found that thereby less impurities bind to the ion-exchange matrix and the ratio of the polypeptide of interest to one or more impurities bound to the matrix during the IEX increases and therefore also in the elution fluid the ratio of the polypeptide of interest to one or more impurities is increased.

In one embodiment the invention relates to a process for the purification of a polypeptide of interest by ion-exchange chromatography wherein a chemical compound is added in a concentration of at least 7 mM
a) to an equilibration fluid of the cation-exchange matrix wherein the equilibration fluid is adjusted such that at least part of the chemical compound in the equilibration fluid binds to the ion-exchange matrix due to a charge that is opposite to the charge of the ion-exchange matrix and/or
b) to a loading fluid which is applied to the ion-exchange matrix and which comprises the polypeptide of interest wherein the loading fluid is adjusted such that at least part of the chemical compound and at least part of the polypeptide of interest in the loading fluid bind to the ion-exchange matrix due to a charge that is opposite to the charge of the ion-exchange matrix and/or
c) to a washing fluid which is used to wash the ion-exchange matrix once the polypeptide of interest has bound to the ion-exchange matrix due to a charge that is opposite to the charge of the ion-exchange matrix wherein the washing fluid is adjusted such that at least part of the chemical compound in the washing fluid binds to the ion-exchange matrix due to a charge that is opposite to the charge of the ion-exchange matrix and that at least part of the polypeptide of interest and at least part of the already bound chemical compound if added at step a) or b) continue to bind to the ion-exchange matrix due to a charge that is opposite to the charge of the ion-exchange matrix
thereby increasing the amount of the polypeptide of interest bound to the ion-exchange matrix relative to the amount of one or more impurities bound to the ion-exchange matrix before the ion-exchange matrix is eluted and thereby leading to an increased ratio of the polypeptide of interest to one or more impurities in the eluate as compared to the same process wherein the chemical compound is added at a concentration of below 7mM.

Preferred concentrations of the chemical compound are least 7mM or at least 10mM, or at least 15mM, or at least 20mM or at least 25mM or at least 30 mM or at least 32mM or at least 40mM or at least 50mM.

The ion-exchange matrix is a cation-exchange matrix.

In a preferred embodiment the positive charge on the chemical compound is clustered.

Processes of the invention reduce impuritities wherein the impurities comprise host cell proteins and/or host cell nucleic acids and/or product-related contaminants and/or viruses and/or prions and/or endotoxins and/or process-related contaminants.

In another embodiment of the invention the polypeptide of interest has during IEX a clustered charge. In preferred embodiments the polypeptide of interest is able to bind a metal ion. In even more preferred embodiments of the invention the polypeptide of interest is a Vitamin K-dependent polypeptide.

The invention also relates to the use of a chemical compound for the reduction of impurities in the purification of a polypeptide of interest by ion-exchange chromatography wherein a chemical compound is added
a) to an equilibration fluid of the ion-exchange matrix wherein the equilibration fluid is adjusted such that at least part of the chemical compound in the equilibration fluid binds to the ion-exchange matrix due to a charge that is opposite to the charge of the ion-exchange matrix
   and/or
b) to a loading fluid which is applied to the ion-exchange matrix and which comprises the polypeptide of interest wherein the loading fluid is adjusted such that at least part of the chemical compound and at least part of the polypeptide of interest in the loading fluid bind to the ion-exchange matrix due to a charge that is opposite to the charge of the ion-exchange matrix
   and/or
c) to a washing fluid which is used to wash the ion-exchange matrix once the polypeptide of interest has bound to the ion-exchange matrix due to a charge that is opposite to the charge of the ion-exchange matrix wherein the washing fluid is adjusted such that at least part of the chemical compound in the washing fluid binds to the ion-exchange matrix due to a charge that is opposite to the charge of the ion-exchange matrix and that at least part of the polypeptide of interest and at least part of the already bound chemical compound if added at step a) or b) continue to bind to the ion-exchange matrix
thereby increasing the amount of the polypeptide of interest bound to the ion-exchange matrix relative to the amount of one or more impurities bound to the ion-exchange matrix before the ion-exchange matrix is eluted thereby leading to an increased ratio of the polypeptide of interest to one or more impurities in the eluate as compared to performing the purification of the polypeptide of interest without adding the chemical compound.

The term **"polypeptide"** as used herein means a polymer made up of five or more amino acids linked together by peptide bonds.

The term **"recombinant polypeptide"** as described herein refers to a polypeptide which is produced by recombinant methods.

The term **"amino acid"** as used herein encompasses both natural and non-naturally occurring amino acids, the latter generally either being capable of being incorporated during recombinant polypeptide synthesis or resulting from post-translational modification.

The term **"purified"** or **"isolate"** means that the polypeptide of interest has been removed from its natural environment or host, and associated impurities reduced or removed such that the molecule in question is the predominant species present (e.g., on a molar basis it is more abundant than any other individual species in the composition/solution). Typically, a composition comprising purified recombinant polypeptide(s) of interest is one where the peptide(s) of interest represents at least 30 percent w/w of all macromolecular species present, preferably at least 50, 60, 70 or 75 percent w/w. A substantially pure composition will comprise more than 80 to 90 percent w/w of recombinant peptide(s) of interest.

**"Polypeptide of interest"** in the sense of the invention refers to a polypeptide for which it is desirable to purify from a mixture according to a method of the present invention. In general such a polypeptide of interest can be commercially sold and requires a certain degree of purity. A preferred type of a polypeptide of interest is a therapeutic polypeptide which can be, for example, a secreted polypeptide. Therapeutic polypeptides include antibodies, antigen-binding fragments of antibodies, soluble receptors, receptor fusions, cytokines, growth factors, enzymes, or clotting factors, some of which are described in more detail herein below. Preferred polypeptides of interest are Vitamin K-dependent polypeptides. The above list of polypeptides is merely exemplary in nature, and is not intended to be a limiting recitation. One of ordinary skill in the art will understand that any polypeptide may be used in accordance with the present invention and will be able to select the particular polypeptide to be produced as needed.

The term **"chromatography"** refers to the process by which a polypeptide of interest, in a mixture is separated from other solutes in the mixture by percolation of the mixture through an adsorbent, which adsorbs or retains a solute more or less strongly due to properties of the solute, such as pi, hydrophobicity, size and structure, under particular buffering conditions of the process. Use of the term "chromatography" includes column and membrane types.

The terms **"ion-exchange" and "ion-exchange chromatography (IEX)"** refer to a chromatographic process in which an ionisable solute of interest (e.g., a polypeptide of interest in a mixture) interacts with an oppositely charged ligand linked (e.g., by covalent attachment) to a solid phase ion exchange material under appropriate conditions of pH and conductivity, such that the solute of interest interacts non-specifically with the charged compound more or less than the solute impurities or contaminants in the mixture. The contaminating solutes in the mixture can be washed from a column of the ion exchange material or are bound to or excluded from the matrix, faster or slower than the solute of interest. "Ion-exchange chromatography" specifically includes anion-exchange chromatography (AEX), cation exchange chromatography (CEX), and mixed mode chromatography where part of the mixed mode is either AEX or CEX.

The phrase **"ion exchange material"** or **"ion exchange matrix"** refers to a solid phase that is negatively charged (i.e. a cation exchange matrix) or positively charged (i.e. an anion exchange matrix). In one embodiment, the charge can be provided by attaching one or more charged ligands (or adsorbents) to the solid phase, e.g. by covalent linking. Alternatively, or in addition, the charge can be an inherent property of the solid phase (e.g. as is the case for silica, which has an overall negative charge). The charge of many ion-exchange matrices is pH dependent and the man skilled in the art can adjust the purification process such that the respective ion-exchange matrix is charged.

A **"buffer"** used in the present invention is a solution that resists changes in pH by the addition of acid or base by the action of its acid-base conjugates components. Various buffers can be employed in a method of the present invention depending on the desired pH of the buffer and the particular step in the purification process [see Buffers. A Guide for the Preparation and Use of Buffers in Biological Systems, Gueffroy, D., ed. Calbiochem Corporation (1975)]. Non-limiting examples of buffer components that can be used to control the pH range desirable for a method of the invention include acetate, citrate, histidine, phosphate, ammonium buffers such as ammonium acetate, succinate, MES, CHAPS, MOPS, MOPSO, HEPES, Tris, and the like, as well as combinations of these TRIS-malic acid-NaOH, maleate, chloroacetate, formate, benzoate, propionate, pyridine, piperazine, ADA, PIPES, ACES, BES, TES, tricine, bicine, TAPS, ethanolamine, CHES, CAPS, methylamine, piperidine, 0-boric acid, carbonic acid, lactic acid, butaneandioic acid, diethylmalonic acid, glycylglycine, HEPPS, HEPPSO, imidazole, phenol, POPSO, succinate, TAPS, amine-based, benzylamine, trimethyl or dimethyl or ethyl or phenyl amine, ethylenediamine, or mopholine. Additional components (additives) can be present in a buffer as needed, e.g., salts can be used to adjust buffer ionic strength, such as sodium chloride, sodium sulfate and potassium chloride; and other additives such as amino acids (such as glycine and histidine), chaotropes (such as urea), alcohols (such as ethanol, mannitol, glycerol, and benzyl alcohol), detergents (see supra.), and sugars (such as sucrose, mannitol, maltose, trehalose, glucose, and fructose). The buffer components and additives, and the concentrations used, can vary according to the type of chromatography practiced in the invention.

Buffers are used in the present invention, including sanitization, equilibration, loading, post- load wash(es), elution or strip buffers. In particular embodiments, a detergent is added to a wash buffer. Examples of detergents that can be used in the invention include, but are not limited to polysorbates (e.g. polysorbates 20 or 80); poloxamers (e.g. poloxamer 188); Triton™; sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g. lauroamidopropyl); myristamidopropyl-, palmidopropyl-, or isostearamidopropyl-dimethylamine; sodium methyl cocoyl-, or disodium methyl oleyl-taurate; MONAQUAT™ series (Mona Industries, Inc., Paterson, N. J.); Igepal CA-630™, Pluronic™, Triton™, BRIJ, Atlas G2127™, Genapol™, HECAMEG™, LUBROL PX™, MEGA™, NP™, THESIT™, TOPPS™, CHAPS, CHAPSO, DDMAU, EMPIGEN BB™, ZWITTERGENT™ and C12E8™. The detergent can be added in any working buffer and can also be included in the feed containing the molecule of interest. Detergents can be present in any amount suitable for use in a polypeptide purification process, e.g., from about 0.001 percent to about 20 percent and typically from about 0.01 percent to about 1 percent. The pH and conductivity of the buffers can vary depending on which step in the purification process the buffer is used. Any suitable buffer at a pH compatible with the selected ligand and matrix/membrane can be used for purifying the polypeptide of interest, such as the buffers described above. In CEX the pH of the buffer can be between about 3 and 10, more preferably from about pH 4.0 to 9.0; conductivity can be from about 0.1 to 40 mS/cm, more preferably from about conductivity 0.5 to 15mS/cm, depending on the purification step and the buffer employed. In AEX the pH of the buffers can be from about 4 to 10, more preferably from about pH 6.0 to 9.0; conductivity can be from about 0.1 to 10.0 mS/cm, more preferably from about 0.5 to 5 mS/cm, depending on the purification step and the buffer employed.

The term **"equilibration fluid"** refers to a liquid which is applied to the ion-exchange matrix before the liquid containing the polypeptide of interest is applied. An **"equilibration fluid"** is used to adjust the pH and conductivity of the ion-exchange matrix prior to loading the matrix with the mixture containing the polypeptide of interest for purification. Suitable buffers that can be used for this purpose are well known in the art, e.g., such as buffers described above, and include any buffer at pH that is compatible with the selected matrix used in the chromatography step for purifying the polypeptide of interest. In particular embodiments, the equilibration buffer species for AEX or CEX are phosphate, carbonate, MES or TRIS based. One preferred buffer for AEX is MES.

The equilibration buffer has a conductivity and/or pH such that the polypeptide of interest is bound to the matrix or such that the polypeptide of interest flows through the column while one or more impurities bind to the column, depending on whether AEX or CEX is used. In a particular embodiment, equilibration is completed when the pH and conductivity of the chromatography medium are within plus or minus 0.2 and plus or minus 0.4 mS/cm of the equilibrating buffer, respectively, more preferably within plus or minus 0.1 and plus or minus 0.2 mS/cm of the equilibrating buffer, respectively. In CEX the pH of the equilibration buffer is from about 3 to about 9, more preferably pH from about 4.0 to about 8.0, conductivity from about 0.1 to about 40 mS/cm, more preferably from about 0.5 to about 10.0 mS/cm. In AEX the pH of the equilibration buffer is from about 4 to about 10, more preferably pH from about 6 to 9, conductivity from about 0.1 (WFI) to about 10 mS/cm, more preferably conductivity from about 0.5 to about 5 mS/cm. When using a POROS® HQ 50 anion-exchange matrix preferred pH ranges are about a pH of 4.5 to 5.5 at a conductivity of about 10 to 20 mS/cm or more preferably about 15- 18 mS/cm respectively.

The term **"loading fluid"** refers to a liquid containing the polypeptide of interest to be isolated and one or more impurities. The loading fluid is passed through the ion-exchange matrix under the operating conditions of the invention. The **"loading fluid"** is used to load the mixture containing the polypeptide of interest onto the column. It shall be appreciated that if a membrane is used as the chromatography medium then the loading fluid is simply contacted with the membrane according to conventional methods used in the art. Any appropriate buffered solution can be used as the loading fluid. In particular embodiments, the buffer used for the loading fluid is a carbonate, a phosphate, a MES or a TRIS buffer. Preferred buffers for the loading fluid for AEX are carbonate or TRIS based. For AEX or CEX, the conductivity and pH of the loading fluid is selected such that the polypeptide of interest is bound to the chromatography medium while most contaminants are able to flow through. Suitable buffers for use as a loading fluid are well known in the art, e.g., such as those described above. It shall be appreciated by those having ordinary skill in the art that loading fluids for AEX or CEX can be used at comparable (if not the same) pH and conductivities as described above for the equilibration buffers for AEX or CEX. When using a POROS® (R) HQ 50 anion-exchange matrix preferred pH ranges are pH 7.0 to 8.0 at a conductivity of 10 to 20 mS/cm or more preferably 15 to 19 mS/cm and more preferably 17 to 19 mS/cm respectively.

The term **"washing fluid"** as used herein, refers to a fluid used to remove impurities from a chromatography matrix (e.g., when using a column) prior to eluting the polypeptide of interest. The term "washing", and grammatical variations thereof, is used to describe the passing of an appropriate washing fluid through or over the chromatography matrix. If desirable, the washing, equilibration and loading fluids can be the same. The pH and conductivity of the washing fluid used in AEX or CEX is such that one or more impurities are eluted from the matrix while the matrix retains the polypeptide of interest. If desirable, the washing fluid can contain a detergent, as described above, such as a polysorbate. It is important to select pH and conductivity of the washing fluid to remove HCPs and other contaminants without significantly eluting the polypeptide of interest. The pH and conductivity of the washing fluid are selected such that the polypeptide of interest is retained in the AEX or CEX matrix used in the process. Examples of buffers suitable for use for a washing fluid are described above. In a particular embodiment, the wash buffer is phosphate, carbonate, MES or TRIS based. A preferred washing buffer in AEX is MES.

The pH of the washing fluid used in AEX or CEX can be from about 3 to about 10, more preferably a pH from about 4 to about 9; and conductivity from about 0.1 to about 40 mS/cm, more preferably from about 3 to about 30 mS/cm. Using a POROS® HQ 50 anion-exchange matrix the pH is preferably between 4.5 to 5.5 and the conductivity is preferentially about 15 to 25 mS/cm and more preferred 21 to 23 mS/cm and even more preferred about 22 mS/cm.

The term **"elution fluid",** as used herein, refers to a buffer used to elute the polypeptide of interest from the AEX or CEX matrix. The terms "elute" and grammatical variations thereof, refers to the removal of a molecule, e.g., polypeptide of interest, from a chromatography material by using appropriate conditions, e.g., altering the ionic strength or pH of the buffer surrounding the chromatography material, by addition of a competitive molecule for the ligand, by altering the hydrophobicity of the molecule or by changing a chemical property of the ligand (e.g. charge), such that the polypeptide of interest is unable to bind the matrix and is therefore eluted from the chromatography column. The pH and conductivity of the elution buffer are selected such that the polypeptide of interest is eluted from the AEX or CEX matrix used in the process. Examples of buffers suitable for use as an elution buffer are described above. In a particular embodiment, the elution buffer is phosphate or TRIS based.

The term **"eluate"** refers to a liquid comprising the polypeptide of interest, which was obtained subsequent to the binding of the polypeptide of interest to a chromatography material and addition of an elution fluid to elute the polypeptide of interest.

The pH of the elution buffer used in AEX or CEX can be from about 3 to about 10, more preferably pH from about 4 to about 9; and conductivity from about 0.1 to about 40 mS/cm, more preferably conductivity from about 5 to about 30 mS/cm. Using a POROS® (R) HQ 50 anion-exchange matrix a preferred pH is between 8.0 and 9.0 and a preferred conductivity is about 17 to 27 mS/cm and more preferred about 22 to 24 mS/cm and even more preferred between about 22.5 to 23 mS/cm.

It is often the case, that the pH must be changed between washing and elution, or later wash buffers or elution buffer may introduce a compound that is not compatible with preceding wash buffers or equilibration buffers. In such circumstances it might be advantageous to introduce a "reequilibrium step" whose purpose is to change the pH of the mobile phase while maintaining the bound state of the protein of interest, or to wash out one compound prior to introduction of its incompatible counterpart.

If desired, additional solutions may be used to prepare the column for reuse. For example, a "regeneration solution" can be used to "strip" or remove tightly bound contaminants from a column used in the purification process. Typically, the regeneration solution has a conductivity and pH sufficient to remove substantially any remaining impurities and polypeptide of interest from the matrix.

In the sense of the invention **"adjust"** means the setting of the operating parameters at a specific step of the IEX. This includes adjusting a certain pH, a certain conductivity, a certain temperature, a certain flow rate and the adjustment of other parameters of an IEX know by the man skilled in the art. The parameters are set such that at least part of the chemical compound in the equilibration fluid and/or the loading fluid and/or the washing fluid binds to the ion-exchange matrix.

The term **"impurity"** in the sense of the invention refers to any foreign or undesirable molecule that is present in a solution such as a loading fluid. An impurity can be a biological macromolecule such as a nucleic acid like a DNA or an RNA, or a polypeptide, other than the polypeptide of interest being purified, that is also present in a sample of the polypeptide of interest being purified. Impurities include, for example, undesirable polypeptide variants, such as aggregated polypeptides, misfolded polypeptides, underdisulfide-bonded polypeptides, high molecular weight species, low molecular weight species and fragments, and deamidated species; other polypeptides from host cells that secrete the polypeptide being purified, host cell DNA, components from the cell culture medium, viruses, prions, endotoxins, process related contaminant which can be molecules that are part of an absorbent used for affinity chromatography that leach into a sample during prior purification steps, for example, Polypeptide A; a nucleic acid; or a fragment of any of the forgoing.

**"Increasing the amount of the polypeptide of interest bound to the ion-exchange matrix relative to one or more impurities bound to the ion-exchange matrix"** means that the ratio of the number of molecules of the polypeptide of interest bound to the ion-exchange matrix to the number of molecules of one or more impurities bound to the ion-exchange matrix is increased versus the ratio of the number of molecules of the polypeptide of interest to the number of molecules of one or more impurities in the loading fluid.

**"Increased ratio of the polypeptide of interest to one or more impurities in the eluate"** means that the ratio of the number of molecules of the polypeptide of interest to the number of molecules of one or more impurities in the eluate is increased versus the ratio of the number of molecules of polypeptide of interest to the number of molecules of one or more impurities in the loading fluid.

**"CHOP Reduction Factor"** means the ratio of the mass of the protein of interest over the mass of CHOP (Chinese Hamster Ovary Cell Proteins) in the elution fluid on the one hand over the mass of the protein of interest over the mass of CHOP in the loading fluid on the other hand. In other words the CHOP Reduction Factor is: (mass_{Protein of Interest}/mass_{CHOP} in the elution fluid) / (mass_{Protein of Interest}/mass_{CHOP} in the loading fluid).

**"Improvement of CHOP Reduction Factor"** means the increase in percent of the CHOP Reduction Factor using a compound according to the invention as compared to the CHOP Reduction Factor when no compound is added in the same purification process under the same conditions as when using the compound. For example the Improvement of CHOP Reduction Factor can be 11 % or 16% or 38 % or 52% or 60%or 89% or 150% or 165% or 223% or 265% or 563% or 1200% or 1325% or 1663% when using different compounds according to the invention with different ion-exchange matrices or with different proteins of interest.

**"CHOP clearance factor"** is the ratio of the mass of CHOP in the loading fluid over the mass of CHOP in the elution fluid.

**"At least in part"** as used here, refers to a certain percentage of the total amount of the chemical compound or the polypeptide of interest which is present in the respective liquid or fluid or is bound to the ion-exchange matrix. This certain percentage is at least 5%, or at least 10%, or at least 20%, or at least 30%, or at least 50%, or at least 60%, or at least 70%, or at least 80%, or at least 90%, or at least 95%, or at least 96%, or at least 97% or at least 98%, or at least 99%. It refers for example to the amount of the chemical compound binding to the ion-exchange matrix when added in the equilibration fluid, or to the amount of the chemical compound or the amount of the polypeptide of interest binding to the ion-exchange matrix when added to the ion-exchange matrix in the loading fluid.

An **"anion exchange matrix"** refers to a solid phase which is positively charged at the time of protein binding, thus having one or more positively charged ligands attached thereto. Any positively charged ligand attached to a solid phase suitable to form the anionic exchange matrix can be used, such as quaternary amino groups. For example, a ligand used in AEC can be a quaternary ammonium, such as quaternary alkylamine and quaternary alky lalkanol amine, or amine, diethylamine, diethylaminopropyl, amino, trimethylammoniumethyl, trimethylbenzyl ammonium, dimethylethanolbenzyl ammonium, and polyamine. Alternatively, for AEC, a membrane having a positively charged ligand, such as a ligand described above, can be used instead of an anion exchange matrix.

Commercially available anion exchange matrices include, but are not limited to, DEAE cellulose, POROS® PI 20, PI 50, HQ 10, HQ 20, HQ 50, D 50 from Applied Biosystems, MonoQ®, MiniQ, Source™ 15Q and 3OQ, Q, DEAE and ANX Sepharose® Fast Flow, Q Sepharose® high Performance, QAE SEPHADEX™ and FAST Q SEPHAROSE® from GE Healthcare, WP PEI, WP DEAM, WP QUAT from J.T. Baker, Hydrocell DEAE and Hydrocell QA from Biochrom Labs Inc., UNOsphere™ Q, Macro-Prep® DEAE and Macro-Prep® High Q from Biorad, Ceramic HyperD® Q, ceramic HyperD® DEAE, Q HyperZ®, Trisacryl® M and LS DEAE, Spherodex® LS DEAE, QMA Spherosil® LS, QMA Spherosil® M from Pall Technologies, DOWEX® Fine Mesh Strong Base Type I and Type II Anion Matrix and DOWEX® MONOSPHER E 77, weak base anion from Dow Liquid Separations, Matrex Cellufine A200, A500, Q500, and Q800, from Millipore, Fractogel® EMD TMAE₃ Fractogel® EMD DEAE and Fractogel® EMD DMAE from EMD, Amberlite™ weak and strong anion exchangers type I and II, DOWEX® weak and strong anion exchangers type I and II, Diaion weak and strong anion exchangers type I and II, Duolite® from Sigma- Aldrich, TSK gel® Q and DEAE 5PW and 5PW-HR, Toyopearl® SuperQ-650S, 650M and 650C₃ QAE-550C and 650S, DEAE-65OM and 650C from Tosoh, and QA52, DE23, DE32, DE51, DE52, DE53, Express-Ion™ D and Express-Ion™ Q from Whatman.

An anion exchange membrane can be used instead of an anion exchange matrix. Commercially available anion exchange membranes include, but are not limited to, Sartobind® Q from Sartorius, Mustang® Q from Pall Technologies and Intercept™ Q membrane from Millipore.

The term **"chemical compound"** refers to chemical compounds which are generally small molecules with a molecular weight of below 1000 Dalton which are charged when used at a certain pH in IEX and which do compete at a certain pH with the polypeptide of interest and with impurities in binding to the ion-exchange matrix. Preferred chemical compounds with the ability to bind metal ions are listed below.

In the sense of the invention the term **"clustered"** means a non-even distribution of the negative charges, meaning that there is a local concentration of negative charges in the chemical compound.

A **"cation exchange matrix"** refers to a solid phase which is negatively charged at the time of protein binding, and which has free cations for exchange with cations in an aqueous solution passed over or through the solid phase. Any negatively charged ligand attached to the solid phase suitable to form the cation exchange matrix can be used, e.g., a carboxylate, sulfonate and others as described below. Commercially available cation exchange matrices include, but are not limited to, for example, those having a sulfonate based group (e.g., MonoS®, MiniS, Source™ 15S and 30S, SP Sepharose® Fast Flow™, SP Sepharose® High Performance from GE Healthcare, Toyopearl® SP-650S and SP-650M from Tosoh, Macro-Prep® High S from BioRad, Ceramic HyperD® S, Trisacryl® M and LS SP and Spherodex® LS SP from Pall Technologies; a sulfoethyl based group (e.g., Fractogel® SE from EMD, POROS® (S-10 and S-20 from Applied Biosystems); a sulphopropyl based group (e.g., TSK Gel® SP 5PW and SP-5PW-HR from Tosoh, POROS® HS-20 and HS 50 from Applied Biosystems); a sulfoisobutyl based group (e.g., Fractogel® EMD SO3 from EMD); a sulfoxyethyl based group (e.g., SE52, SE53 and Express-Ion™ S from Whatman), a carboxymethyl based group (e.g., CM Sepharose® Fast Flow from GE Healthcare, Hydrocell CM from Biochrom Labs Inc., Macro-Prep® CM from BioRad, Ceramic HyperD® CM, Trisacryl M CM, Trisacryl LS CM, from Pall Technologies, Matrex Cellufine C500 and C200 from Millipore, CM52, CM32, CM23 and Express - Ion™ C from Whatman, Toyopearl® CM-650S, CM-650M and CM-650C from Tosoh); sulfonic and carboxylic acid based groups (e.g. BAKERBOND® Carboxy-Sulfon from J.T. Baker); a carboxylic acid based group (e.g., WP CBX from J.T Baker, DOWEX® MAC-3 from Dow Liquid Separations, Amberlite™ Weak Cation Exchangers, DOWEX® Weak Cation Exchanger, and Diaion Weak Cation Exchangers from Sigma- Aldrich and Fractogel® EMD COO- from EMD); a sulfonic acid based group (e. g., Hydrocell SP from Biochrom Labs Inc., DOWEX® Fine Mesh Strong Acid Cation Matrix from Dow Liquid Separations, UNOsphere® S, WP Sulfonic from J. T. Baker, Sartobind® S membrane from Sartorius, Amberlite™ Strong Cation Exchangers, DOWEX® Strong Cation and Diaion Strong Cation Exchanger from Sigma-Aldrich); and a orthophosphate based group (e.g., PI 1 from Whatman). If desirable, a cation exchange membrane can be used instead of a cation exchange matrix, e.g., Sartobind® S (Sartorius; Edgewood, NY).

The term **"ability to complex metal ions"** means the ability for the formation of a complex between the chemical compound or the polypeptide of interest and a metal ion. The formation of a complex encompasses the formation of two or more separate bindings between a polydentate ligand, i.e. the chemical compound or the polypeptide of interest and a single central atom. Usually such chemical compounds are organic chemical compounds, and are called chelants, chelators, chelating agents, or sequestering agents. The chemical compound forms a chelate complex with the single central atom, which may be a metal ion. Chelate complexes are contrasted with coordination complexes with monodentate ligands, which form only one bond with the central atom. By way of non-limiting examples the following agents are chemical compounds with the ability to complex metal ions according to the invention:
a) chemical compounds comprising a penta-acetic group and their various salts, like diethylene triamine pentaacetic acid (DTPA or pentetic acid), Pentetide, Ino-1 and Fura-2;
b) chemical compounds comprising a tetra-acetic group and their various salts, like 1,2-bis(o-ethane-N,N,N',N'-tetra-acetic acid (BAPTA), ethylene diamine tetra-acetic acid (EDTA) and its various salts (like diammonium EDTA, dipotassium EDTA dihydrate; disodium EDTA; trisodium EDTA; tetrasodium EDTA and tetraammonium EDTA) and ethylene glycol tetra-acetic acid (EGTA);
c) chemical compounds comprising a tri-acetic group and their various salts, like N-(Hydroxyethyl) ethylene diamine tri-acetic acid (HEDTA) and nitrilotriacetic acid (NTA);
c) chemical compounds comprising a tri-acetic group and their various salts, like N-(Hydroxyethyl) ethylene diamine tri-acetic acid (HEDTA) and (NTA);
d) chemical compounds comprising a di-acetic group and their various salts like Imino diacetic acid (IDA), tetrasodium iminodisuccinate, trisodiumcitrate and
e) chemical compounds comprising multiple amine groups, like aminoethyl-ethanolamine (AEEA), 2,3-diphenylethylenediamine, ethylen-diamine, ethylenediamine-N,N'-bis(2-hydroxyphenylacetic acid) ethylene-diamine-*N*,*N'-*disuccinic acid, , tetrahydroxypropyl ethylenediamine, triethylenetetramine and polyaminocarboxylic acid
f) chemical compounds comprising multiple thiol groups, like dimeracprol, dimercaptosuccinic acid (DMSA), dimercapto-1-propanesulfonic acid (DMPS), sodium diethyldithiocarbamate
g) phosponic acid and derivatives thereof and their various salts, like aminotris(methylenephosphonic acid) (ATMP), diethylenetriamine penta(methylene phosphonic acid), ethylenediamine tetra(methylene phosphonic acid (EDTMP), etidronic acid or 1-hydroxyethane 1,1-diphosphonic acid (HEDP).

It is to be understood that listing a certain chemical compound as an ion or a certain salt encompasses the ion in different salts as well.

Also the following complexing agents are chemical compound with the ability to complex metal ions in the sense of the invention. Acetylacetonic acid, Acetylacetone, Benzotriazole, 2,2'-Bipyridine, 4,4'-Bipyridine, 1,2-Bis(dimethyl-arsino)benzene 1,2 Bis(dimethyl-phosphino)ethane, 1,2-Bis(diphenylphosphino)-ethane, Benzotriazoles, Clathro-chelate, 2.2.2-Cryptand, Catechol, Corrole, Crown ether, 18-Crown-6, Cryptand, Cyclen, Cyclodextrins, Deferasirox, Deferiprone, Deferoxamine, Dexrazoxane, Diglyme, Dimethylglyoxime, Dithiolene, Ethandiol, Etidronic acid, Ferrichrome, Gluconic acid, Metallacrown, Hydrolyzed casein, Hexafluoroacetylacetone, Penicillamine, Phenanthroline, Phosphonate, Phytochelatin, Porphin, Porphyrin, Pyrophosphate, Scorpionate ligand, Sodium poly(aspartate), Terpyridine, Tetraphenyl-porphyrin, 1,4,7-Triazacyclononane, Trimetaphosphates Triphos, and 1,4,7-Trithiacyclononane.

EDTA is a polyamino carboxylic acid with the formula [CH2N(CH2CO2H)2]2. Its usefulness arises because of its role as a chelating agent, i.e. its ability to "sequester" metal ions such as Ca²⁺ and Fe³⁺. After being bound by EDTA, metal ions remain in solution but exhibit diminished reactivity. EDTA is produced as several salts, notably disodium EDTA and calcium disodium EDTA. The molecular weight is 292.24 Da. In coordination chemistry, EDTA⁴⁻ is a member of the polyamino carboxylic acid family of ligands. EDTA⁴⁻ usually binds to a metal cation through its two amines and four carboxylates. Many of the resulting coordination chemical compounds adopt octahedral geometry. EDTA is also capable to bind to cationic charges of AEX matrices.

Processes use the chemical compound like EDTA in a concentration between 7mM and a concentration where the conductivity the chemical compound introduces to the equilibration fluid or the sample fluid or the wash fluid is so high that the binding of the polypeptide of interest no longer takes place. This maximal concentration of the chemical amount can easily be determined by the man skilled in the art for each individual combination of a given chemical compound and a given polypeptide of interest. For EDTA the preferred concentration for processes are at least 7mM or at least 10mM, or at least 15mM, or at least 20mM or at least 25mM or at least 30 mM or at least 32mM or at least 40mM or at least 50mM. For EDTA the range at which the conductivity of the equilibration fluid, or the sample fluid or the wash fluid becomes too high for efficient binding of the polypeptide of interest starts at about 200mM. So preferred processes is the use up to 200mM EDTA, or up to 190mM, or up to 180mM or up to 170mM or up to 160mM or up to 150mM or up to 140mM or up to 130mM. It is to be understood the disclosed lower ranges for the concentration of EDTA can be combined with the disclosed upper concentration to form preferred ranges of concentration, like 7mM to 200mM, 7mM to 190mM, 7mM to 180mM...... or 10mM to 200mM, or 10mM to 190mM, or 10 mM to 180mM........., or 15mM to 200mM or 15mM to 190mM or 15mM to 180mM....

Also especially preferred are processes using Ethylene glycol tetra-acetic acid (EGTA) as the chemical compound.

Examples for anionic compounds which can be used according to the invention in the purification of a polypeptide of interest by CEX are primary amines and cationic amino acid polymers, such as tetraethylene pentamine (TEPA), dipicolylamine (DPA), poly-lysine, poly-arginine and poly-histidine.

Preferred polypeptides to be purified by processes of the invention are charged and have preferentially clustered charges or do comprise functional groups as listed above for the chemical compounds, like amine or thiol groups, which are able to contribute to a strong binding to the ion-exchange matrix. Preferentially these functional groups are also clustered.

The term **"host cell polypeptide",** or **"HCP",** refers to any of the polypeptides derived from the metabolism (intra and extra-cellular) of the host cell that expresses the target polypeptide, including any polypeptides expressed from the genome of the host cell or polypeptides that are recombinantly expressed, and which are not considered the target polypeptide. The host cell can be any cell that is capable of expressing the target polypeptide, particularly mammalian (e.g., CHO and murine myeloma cell lines such as NSO), insect bacterial, plant and yeast cell lines. In a particular embodiment of the invention, the HCP is a "Chinese hamster ovary cell polypeptide", or "CHOP", which refers to any of the host cell polypeptides ("HCP") derived from a Chinese hamster ovary ("CHO") cell culture. The HCP is present generally as an impurity in a cell culture medium or lysate [(e.g., a harvested cell culture fluid ("HCCF")], which contains the polypeptide of interest. The amount of HCP present in a mixture comprising a polypeptide of interest provides a measure of the degree of purity for the polypeptide of interest. Typically, the amount of HCP in a polypeptide mixture is expressed in parts per million relative to the amount of the polypeptide of interest in the mixture. Accordingly, in some embodiments, an eluate containing a product has HCPs present in less than 100ppm, or less than 90ppm, or less than 80ppm, or less than 70ppm, or less than 60ppm, or at less than 50ppm, or less than 40ppm, or less than 30ppm, or less than 20ppm, or less than 10ppm or less than 5ppm.

HCP composition is extremely heterogeneous and dependent on the polypeptide product and purification procedure used. Prior to any marketing approval of a biological product for therapeutic use, the level of contaminating polypeptides (such as HCPs) in the product must be quantitatively measured according to the ICH and FDA guidelines.

Host cell polypeptides as discussed above can be human polypeptides if a human cell line is used for production of the polypeptide of interest or non-human polypeptides, if a non-human cell line is used for production of the polypeptide of interest. In one aspect of the invention, the polypeptide contaminant is a host cell polypeptide, such as a Vitamin K-dependent polypeptide. A particularly relevant class of host cell polypeptides are Gla-domain containing polypeptides such as GAS-6, Polypeptide S, Factor II (Prothrombin), thrombin, Factor X/Xa, Factor IX/IXa, Polypeptide C, Factor VII/VIIa, Polypeptide Z, Transmembrane gamma-carboxyglutamic acid polypeptide 1, Transmembrane gamma-carboxyglutamic acid polypeptide 2, Transmembrane gamma carboxyglutamic acid polypeptide 3, Transmembrane gamma-carboxyglutamic acid polypeptide 4, Matrix Gla polypeptide, and Osteocalcin.

As mentioned above, the Vitamin K-dependent polypeptide of interest is typically a Vitamin K-dependent coagulation factor selected from Factor VII polypeptides, Factor IX polypeptides, Factor X polypeptides and activated Polypeptide C. In one more particular embodiment, the Vitamin K-dependent polypeptide of interest is a Factor IX polypeptide. In another more particular embodiment, the Vitamin K-dependent polypeptide of interest is a Factor VII polypeptide. In another particular embodiment, the Vitamin K-dependent polypeptide of interest is a Factor X polypeptide.

In another embodiment, the invention provides a method for separation of non-human polypeptide contaminants from human Vitamin K-dependent polypeptide. In one aspect of the invention, the non-human polypeptide contaminants are also Vitamin K-dependent polypeptides. In yet another aspect, the non-human polypeptide contaminants are hamster polypeptides.

The term **"parts per million"** or **"ppm"** are used interchangeably herein to refer to a measure of purity of the polypeptide of interest purified by a method of the invention. The units ppm refer to the amount of HCP in nanograms/milliliter per polypeptide of interest in milligrams/milliliter, where the polypeptides are in solution (i.e., as described in an Example infra, HCP ppm=(CHOP ng/ml)/(polypeptide of interest mg/ml)). Where the polypeptides are dried, such as by lyophilization, ppm refers to (HCP ng)/(polypeptide of interest mg).

There are different methods for determining host cell protein (HCP) levels. Failure to identify and sufficiently remove HCPs from the polypeptide of interest may lead to reduced efficacy and/or adverse patient reactions. One method is a "HCP ELISA" referring to an ELISA where the second antibody used in the assay is specific to the HCPs produced from cells, e.g., CHO cells, used to generate the antibody. The second antibody may be produced according to conventional methods known to those of skill in the art. For example, the second antibody may be produced using HCPs obtained by sham production and purification runs, i.e., the same cell line used to produce the antibody of interest is used, but the cell line is not transfected with antibody DNA. In an exemplary embodiment, the second antibody is produced using HCPs similar to those expressed in the cell expression system of choice, i.e., the cell expression system used to produce the target antibody.

Generally, HCP ELISA comprises sandwiching a liquid sample comprising HCPs between two layers of antibodies, i.e., a first antibody and a second antibody. The sample is incubated during which time the HCPs in the sample are captured by the first antibody, e.g., goat anti-CHO, affinity purified (Cygnus). A labeled second antibody specific to the HCPs produced from the cells used to generate the antibody, e.g., anti- CHO HCP Biotinylated, is added, and binds to the HCPs within the sample. The amount of HCP contained in the sample is determined using the appropriate test based on the label of the second antibody. HCP ELISA may be used for determining the level of HCPs in an antibody composition, such as an eluate or flowthrough obtained using the process described in section III above. The present invention also provides a composition comprising an antibody, wherein the composition has no detectable level of HCPs as determined by an HCP Enzyme Linked Immunosorbent Assay ("ELISA").

The term **"host cell nucleic acids"** means any polynucleotides e.g. RNA or DNA which were present in the host cell expressing the polypeptide of interest. A nucleic acid molecule may be single-stranded or double-stranded.

The term **"product related contaminants"** relates to degradation products, aggregates or misfolded or otherwise denatured forms of the polypeptide of interest, which are undesirable and should be removed from the final pure polypeptide of interest. One degradation product of particular interest which can be reduced is FIXalpha in the purification of FIX.

The term **"viruses"** does include DNA or RNA viruses. These viruses may be enveloped or non-enveloped by a phospholipid membrane.

The term **"prions"** relates to transmissible misfolded proteins. These misfolded proteins can cause the correctly folded versions of the protein to themselves become misfolded.

The term **"endotoxins"** means lipopolysaccharides derived from cell membranes.

The term **"process related impurities"** relates to any molecules especially proteins added in the process. By way of non-limiting example such process related impurities can be Protein A, polysorbate-80, tri-n-Butyl phosphate or leached ligands e.g. from monoclonal antibodies from an upstream immunoaffinity purification.

The term **"harvested cell culture fluid",** means prokaryotic or eukaryotic cell culture fluid from which the cells have been removed, by means including centrifugation or filtration. Cell culture is the process by which either prokaryotic or eukaryotic cells are grown under controlled conditions.

The term **"cell culture"** refers to the culturing of cells derived from multicellular eukaryotes, including animal cells or monocellular prokaryotes, including bacteria and yeast. Eukaryotic cell cultures include mammalian cells such as Chinese Hamster Ovary cells, hybridomas, and insect cells. With an appropriate cell culture vessel, secreted polypeptides can be obtained from anchorage dependent cells or suspension cell lines. Mammalian cell cultures include Chinese Hamster Ovary (CHO) cells.

The term **"reduced"** refers to the lessening or diminishing the amount of a substance. A reduced preparation includes a preparation which has less of a substance, such as HCPs, relative to an initial amount. In one embodiment, the substance is an impurity or contaminant. In one embodiment, the term "reduced" means substantially less of the substance. In another embodiment, the term "reduced" means no amount of the substance. In one embodiment, no amount of a substance includes "no detectable amount" using assays described herein.

The term **"substantially free"** includes no amount of a substance, but can also include a minimal amount of a substance. In one embodiment, no amount of a substance includes "no detectable amount" using assays described herein.

The term **"detergent"** refers to ionic, zwitterionic and nonionic surfactants, which are useful for preventing aggregation of polypeptides and to prevent non-specific interaction or binding of contaminants to the polypeptide of interest, and can be present in various concentrations.

A **"sanitization"** solution is typically used to clean the matrix used in column chromatography by removing any bound contaminants, e.g., those of biological origin, prior to the purification process. Any desirable buffer could be used for this purpose provided it is compatible with the particular column and matrix selected according to a method of the invention. Preferably, the pH of the sanitization solution is high, e.g., pH 10 or greater, more preferably pH 1 1 or greater, and still more preferably pH 12 or greater; alternatively, the pH of the sanitization solution can be low, e.g. pH 4 or less, more preferably pH 3 or less. In a particular embodiment, a matrix used in a method of the invention is cleaned using a sanitization solution that includes IN NaOH, pH greater than 12.

As used herein, the term **"conductivity"** refers to the ability of an aqueous solution to conduct an electric current between two electrodes at a particular temperature. A current flows by ion transport in solution. Therefore, with an increasing amount of ions present in the aqueous solution, the solution will have a higher conductivity. In a method of the present invention the temperature at which purification is typically performed can be from about 4 to about 37 degrees centigrade, more preferably from about 15 to about 25 degrees centigrade within the specified pH ranges.

The unit of measurement for conductivity is milliSiemens per centimeter (mS/cm), and can be measured using a standard conductivity meter. The conductivity of a solution can be altered by changing the concentration of ions therein. For example, the concentration of a buffering agent and/or concentration of a salt (e.g. NaCl or KCl) in the solution may be altered in order to achieve the desired conductivity. Preferably, the salt concentration is modified to achieve the desired conductivity as described in the Example below. The conductivity of the respective fluid is not an essential feature of the invention. The man skilled in the art can determine with standard experiments conductivities in the equilibration fluid, the loading fluid or the elution fluid which allow both the polypeptide of interest and the chemical compound to bind to the ion-exchange matrix.

The **"pI" or "isoelectric point"** of a polypeptide refers to the pH at which the polypeptide's positive charge balances its negative charge. The pI can be calculated according to various conventional methodologies, e.g., from the net charge of the amino acid and/or sialic acid residues on the polypeptide or by using isoelectric focusing.

### Vitamin K-dependent polypeptides of interest

The present invention relates in a broad aspect to the purification of a Vitamin K-dependent polypeptide of interest and to particular purified compositions comprising such polypeptides. The term "of interest" is applied herein as a pointer to the particular species (a Vitamin K-dependent polypeptide) which is relevant to obtain in the most pure form, e.g. for the purpose of using the Vitamin K-dependent polypeptide in a therapeutic context.

The methods described herein may in principle be applicable to the purification of any Vitamin K-dependent polypeptide comprising, but not limited to, GAS-6, Polypeptide S, Factor II (Prothrombin), Thrombin, Factor X/Xa, Factor IX/IXa, Polypeptide C, Factor VII/VIIa, Polypeptide Z, Transmembrane gamma-carboxyglutamic acid polypeptide 1, Transmembrane gamima- carboxyglutamic acid polypeptide 2, Transmembrane gamma carboxyglutamic acid polypeptide 3, Transmembrane gamma-carboxyglutamic acid polypeptide 4, Matrix Gla polypeptide, and Osteocalcin), in particular Vitamin K-dependent coagulation factors selected from Factor VII polypeptides, Factor IX polypeptides, Factor X polypeptides and activated Polypeptide C. In one particular embodiment, the method is used for the purification of recombinant Vitamin K- dependent polypeptides of interest produced under cell culture conditions, in particular non-human cell cultures.

In one particular embodiment, the Vitamin K-dependent polypeptide of interest is a Factor IX polypeptide, such as FIX or FIXa.

As used herein, the terms "Factor IX polypeptide" and "FIX polypeptide" means any polypeptide comprising the amino acid sequence of wild-type human Factor IX (i.e., a polypeptide having the amino acid sequence disclosed in U.S. Patent No. 4,994,37 or European Patent No. EP 0107278), variants thereof as well as Factor IX-related polypeptides, Factor VII derivatives and Factor VII conjugates. This includes Factor IX variants, Factor IX-related polypeptides, Factor IX derivatives and Factor IX conjugates exhibiting substantially the same or improved biological activity relative to wild-type human Factor IX.

Human FIX, a member of the group of vitamin K-dependent polypeptides, is a single-chain glycoprotein with a molecular weight of 57 kDa, which is secreted by liver cells into the blood stream as an inactive zymogen of 415 amino acids. It contains 12 γ-carboxy-glutamic acid residues localized in the N-terminal Gla-domain of the polypeptide. The Gla residues require vitamin K for their biosynthesis. Following the Gla domain there are two epidermal growth factor domains, an activation peptide, and a trypsin-type serine protease domain. Further posttranslational modifications of FIX encompass hydroxylation (Asp 64), N-(Asn157 and Asn167) as well as O-type glycosylation (Ser53, Ser61, Thr159, Thr169, and Thr172), sulfation (Tyr155), and phosphorylation (Ser158).

FIX is converted to its active form, Factor IXa, by proteolysis of the activation peptide at Arg145-Ala146 and Arg180-Val181 leading to the formation of two polypeptide chains, an N-terminal light chain (18 kDa) and a C-terminal heavy chain (28 kDa), which are held together by one disulfide bridge. Activation cleavage of Factor IX can be achieved in vitro e.g. by Factor XIa or Factor VIIa/TF. Factor IX is present in human plasma in a concentration of 5-10 µg/ml. Terminal plasma half-life of Factor IX in humans was found to be about 15 to 18 hours (White GC et al. 1997. Recombinant factor IX. Thromb Haemost. 78: 261-265; Ewenstein BM et al. 2002. Pharmacokinetic analysis of plasma-derived and recombinant F IX concentrates in previously treated patients with moderate or severe hemophilia B. Transfusion 42:190-197).

In another embodiment, the invention relates to methods for separation of a full length FIX from FIXalpha. FIXalpha is one specific proteolytic breakdown product resulting from cleavage of FIX at only the Arg145 - Ala146 activation site. This form of protein is not separable by SEC, as it remains together via disulphide bonds and is thus a specific problem in the purification of FIX. In one embodiment of the invention the amount of FIXalpha is reduced versus the amount of intact FIX by using processes of the invention.

In another particular embodiment, the Vitamin K-dependent polypeptide of interest is a Factor VII polypeptide, such as a Factor VII-related polypeptide, or a Factor VII derivatives, or a Factor VII conjugate, in particular a human Factor VII polypeptide, in particular human wild type Factor VII or wild type human Factor VIIa.

As used herein, the terms "Factor VII polypeptide" and "FVII polypeptide" means any polypeptide comprising the amino acid sequence 1-406 of wild-type human Factor VIIa (i.e., a polypeptide having the amino acid sequence disclosed in U.S. Patent No. 4,784,950), variants thereof as well as Factor VII-related polypeptides, Factor VII derivatives and Factor VII conjugates. This includes Factor VII variants, Factor VII-related polypeptides, Factor VII derivatives and Factor VII conjugates exhibiting substantially the same or improved biological activity relative to wild-type human Factor Vila.

FVII is a single-chain glycoprotein with a molecular weight of about 50 kDa, which is secreted by liver cells into the blood stream as an inactive zymogen of 406 amino acids. It contains 10 γ-carboxy-glutamic acid residues (positions 6, 7, 14, 16, 19, 20, 25, 26, 29, and 35) localized in the N-terminal Gla-domain of the protein. The Gla residues require vitamin K for their biosynthesis. Located C-terminal to the Gla domain are two epidermal growth factor domains followed by a trypsin-type serine protease domain. Further posttranslational modifications of FVII encompass hydroxylation (Asp 63), N- (Asn145 and Asn322) as well as O-type glycosylation (Ser52 and Ser60).

FVII is converted to its active form Factor Vila by proteolysis of the single peptide bond at Arg152-lle153 leading to the formation of two polypeptide chains, a N-terminal light chain (24 kDa) and a C-terminal heavy chain (28 kDa), which are held together by one disulfide bridge. In contrast to other vitamin K-dependent coagulation factors no activation peptide, which is cleaved off during activation of these other vitamin-K dependent coagulation factors has been described for FVII. The Arg152-lle153 cleavage site and some amino acids downstream show homology to the activation cleavage site of other vitamin K-dependent polypeptides.

### Recombinant Expression

Recombinant production of polypeptides can be achieved using various techniques. Typically the polynucleotide sequence of interest is cloned into an "expression vector". The vector may be a plasmid vector, a viral vector, or any other suitable vehicle adapted for the insertion of foreign sequences, their introduction into eukaryotic or prokaryotic cells and the expression of the introduced sequences as appropriate. Typically the vector includes transcriptional/translational control sequences required for expression of the fusion polypeptide in a host cell, such as a promoter, a ribosome binding site, an initiation codon, a stop codon, optionally an operator sequence and possibly other regulatory sequences such as enhancers.

The recombinant expression vectors suitable for producing the recombinant polypeptides of the invention typically include one or more regulatory sequences, selected on the basis of the host cells to be used for expression, operably linked to the nucleic acid sequence encoding the fusion polypeptide to be expressed. It will be appreciated by those skilled in the art that the design of the expression vector can depend on such factors as the choice of the host cell to be transformed and the level of expression of peptide desired. Vector DNA can be introduced into prokaryotic or eukaryotic cells via conventional transformation or transfection techniques. Methods and materials for preparing recombinant vectors, transforming host cells using replicating vectors, and expressing biologically active foreign polypeptides are generally well known in the art. The recombinant polypeptides of the invention may also be produced in a cell-free system.

The recombinant polypeptides of interest may also be designed so that they are secreted extracellularly and/or transported to specific locations in the cell. For example, the recombinant peptide may be designed to be transported to an organelle, such as plastid or vacuole in a plant cell or other type of non- membrane cellular body or inclusion such as those that exist in prokaryotes. Methods employed to target peptides to extra-cellular or cellular locations are generally known in the art. For example, recombinant peptides and/or vector constructs may be designed to include targeting sequence(s) and/or post- translation modifications that enable cellular transport. Such transport may be post-translational or co-translational.

The recombinant polypeptides of interest may also be produced in a particular organelle or cellular location, such as a plastid. Appropriate methods for achieving expression in the desired location are known to those of skill in the art and involve, for example design of the recombinant peptide and/or vectors with suitable promoters and other 5' and 3' control sequences as required.

In addition to prokaryotes, eukaryotic microbes such as filamentous fungi or yeast are suitable cloning or expression hosts for polypeptide encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e.g., K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderraa reesia (EP 244,234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger. Suitable host cells for the expression of glycosylated antibodies are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e.g., the L-I variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

Preferred mammalian host cells for expressing the recombinant antibodies of the invention include Chinese Hamster Ovary (CHO cells) (including dhfr- CHO cells, described in Urlaub and Chasin, (1980) PNAS USA 77:4216-4220, used with a DHFR selectable marker, e.g., as described in Kaufman and Sharp (1982) Mol. Biol. 159:601- 621), NSO myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Other examples of useful mammalian host cell lines are monkey kidney CVI line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse Sertoli cells (TM4, Mather, Biol. Reprod. 23:243-251 (1980)); monkey kidney cells (CVI ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N. Y. Acad. Sci. 383:44-68 (1982)); MRC 5 cells; FS4 cells; and a human hepatoma line (Hep G2).

Host cells are transformed with the above-described expression or cloning vectors for production and cultured in conventional nutrient media modified as appropriate for inducing promoters, selecting transformants, or amplifying the genes encoding the desired sequences.

### Pharmaceutical compositions

Polypeptides obtained using the process of the invention may be incorporated into pharmaceutical compositions suitable for administration to a subject. Typically, the pharmaceutical composition comprises an antibody, or antigen-binding portion thereof, and a pharmaceutically acceptable carrier.

As used herein, **"pharmaceutically acceptable carrier"** includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it is preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of antibody, or antigen-binding portion thereof.

Polypeptides of interest obtained using the process of the invention may be incorporated into pharmaceutical compositions suitable for administration to a subject. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it is preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of antibody, or antigen-binding portion thereof.

The solution may be treated to remove some or all of the solvent e.g. by freeze drying, spray-drying, lyophilisation, or to otherwise recover the recombinant peptide from the solution. The recombinant peptide may then be stored, for example, as a liquid formulation or solid preparation. The desired formulation of the end product will be determined by the required downstream application of the peptide.

Pharmaceutical compositions comprising polypeptides of the invention may be found in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions. The preferred mode of administration is parenteral (e.g., intravenous, subcutaneous, intraperitoneal, intramuscular). In a preferred embodiment, the antibody is administered by intravenous infusion or injection. In another preferred embodiment, the antibody is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (i.e., antibody, or antigen-binding portion thereof) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The polypeptides obtained using the methods of the present invention can be administered by a variety of methods known in the art, although for many therapeutic applications, the preferred route/mode of administration is subcutaneous injection. In another embodiment, administration is via intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art.

The pharmaceutical compositions may include a **"therapeutically effective amount"** or a **"prophylactically effective amount"** of a polypeptide of interest purified using a process according to the invention. A "therapeutically effective amount" refers to an amount effective, at dosages and for periods of time necessary, to achieve the desired therapeutic result. A therapeutically effective amount of the antibody, or antigen- binding portion thereof, may vary according to factors such as the disease state, age, sex, and weight of the individual, and the ability of the polypeptides to elicit a desired response in the individual. A therapeutically effective amount is also one in which any toxic or detrimental effects of the antibody, or antigen- binding portion thereof, are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount. Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit comprising a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

Also disclosed herein are packaged pharmaceutical compositions, articles of manufacture, or kits comprising the antibody, or antigen-binding portion thereof, obtained using the process of the invention. The article of manufacture may comprise an antibody, or antigen-binging portion thereof, obtained using the method of the invention and packaging material. The article of manufacture may also comprise label or package insert indicating the formulation or composition comprising the antibody, or antigen- binding portion thereof, has a reduced level of HCP.

The present invention will now be further described with reference to the following examples. The examples refer to figures:

### Description of the figures

- Figure 1:: CHOP clearance at various EDTA concentrations
- Figure 2:: Analytical SEC of the eluate from the control and EDTA-containing processes
- Figure 3:: Factor IX alpha level in the eluate of control and EDTA-containing processes
- Figure 4:: CHOP clearance in large scale manufacturing
- Figure 5:: CHOP Reduction Factor for different compounds on POROS® 50 HQ.
- Figure 6:: CHOP Reduction Factor for different anion exchange matrices using EDTA.
- Figure 7:: CHOP Reduction Factor for POROS® 50 HQ and EDTA only in post-load wash buffer.
- Figure 8:: CHOP Reduction Factor for monoclonal antibody on anion exchange matrix Fractogel® EMD TMAE (M) using EDTA.
- Figure 9:: CHOP Reduction Factor for monoclonal antibody on cation exchange matrix Fractogel® EMD SO3 (M) using DPA.
- Figure 10:: CHOP reduction factors for rVlla-FP on POROS® 50 HQ using different concentrations of EDTA versus control without EDTA addition

### Examples

### Reference Example 1 : Method for EDTA-mediated purification of rIX-FP on POROS® - HQ

rIX-FP was expressed as an albumin fusion protein in CHO cells with FIX fused at its C-terminus to human albumin as described in WO2007/144173. After fermentation the culture was subjected to filtration through a Cuno Zeta 60SP followed by 10SP. The clarified cell culture fluid was then processed either via the "EDTA-containing" anion exchange method according to the invention or via the "control" method.

### Example 1a: Control method

The clarified cell culture fluid (loading fluid) was applied to an anion exchange column packed with POROS® HQ 50 (Applied Biosystems) at 380 cm/hr. This column had previously been equilibrated by applying three column volumes of an equilibration fluid (50mM MES, 150mM NaCl, pH5.0) at 380 cm/hr. The conductivity was in the range of 16 to 18 mS/cm. After applying the clarified cell culture fluid, any unbound material was washed from the column by applying three column volumes a washing fluid (50mM MES, 150mM NaCl, pH 5.0) at 380 cm/hr. Further contaminants, less strongly bound than the rIX-FP, were then washed from the column by applying five column volumes of a washing fluid (50mM MES, 195mM NaCl, 2mM CaCl₂, pH 5.0) at 380 cm/hr, followed by re-equilibration with 2 column volumes of 50mM Tris-HCl, 100mM NaCl, pH 8.5 at 380 cm/hr preventing loss of rIX-FP protein that would result from the introduction of CaCl₂ while the high conductivity of the wash buffer was present on the column or something to that effect and finally 5 column volumes of 50mM Tris-HCl, 100mM NaCl, 10mM CaCl₂, pH 8.5 at 380 cm/hr. The purified rIX-FP was then eluted from the column by applying 5 column volumes of an elution fluid having 50mM Tris-HCl, 150mM NaCl, 30mM CaCl₂, pH 8.5 at 190 cm/hr.

Following elution and collection of the purified rIX-FP, the column was regenerated by applying 3 column volumes of 50mM Tris-HCl, 2M NaCl, pH 8.5 at 380 cm/hr.

### Example 1b: EDTA-containing method

To the clarified cell culture fluid EDTA was added to the level under investigation (see Table 1) or to a value in the range of 5 to 150mM. The conductivity was then adjusted to ≤18 mS/cm to give equivalence regarding the conductivity to the control sample with a dilution buffer consisting of 20mM Tris, pH 7.0 plus EDTA at the same concentration as it was added to the cell culture fluid.

When doing large scale preparations at 35 mM EDTA (see Example 1f) the clarified cell culture fluid was first adjusted to a conductivity of ≤ 13.5 mS/cm by dilution with 20mM Tris-HCl, pH7.0. Disodium-EDTA was then added to this solution to a level of 35mM bringing the conductivity back up to a level comparable to that of the unadjusted control feedstock (≤18 mS/cm).

Either way the final solution (loading fluid) was applied to an anion exchange column packed with POROS® HQ 50 (Applied Biosystems) at 380 cm/hr. This column had previously been equilibrated by applying three column volumes of an equilibration fluid (50mM MES, 100mM NaCl, 50mM disodium-EDTA, pH5.0) at 380 cm/hr. The conductivity of this equilibration buffer was similar to that of the corresponding buffer in the control process by decreasing the NaCl concentration.

After applying the adjusted, EDTA containing clarified cell culture fluid (loading fluid), any unbound material was washed from the column by applying three column volumes of a washing fluid (50mM MES, 100mM NaCl, 50mM disodium-EDTA, pH5.0) at 380 cm/hr. Further contaminants, less strongly bound than the rIX-FP, were then washed from the column by applying five column volumes of a washing fluid (50mM MES, 195mM NaCl, 2mM CaCl₂, pH5.0) at 380 cm/hr followed by re-equilibration with 2 column volumes of 50mM Tris-HCl, 100mM NaCl at 380 cm/hr, pH8.5 and finally 5 column volumes of 50mM Tris-HCl, 100mM NaCl, 10mM CaCl₂, pH8.5 at 380 cm/hr. The purified rIX-FP was then eluted from the column by applying 5 column volumes of an elution fluid (50mM Tris-HCl, 150mM NaCl, 30mM CaCl₂, pH8.5) at 190 cm/hr.

Following elution and collection of the purified rIX-FP, the column was regenerated by applying 3 column volumes of 50mM Tris-HCl, 2M NaCl, pH8.5 at 380 cm/hr.

### Example 1c: CHOP clearance

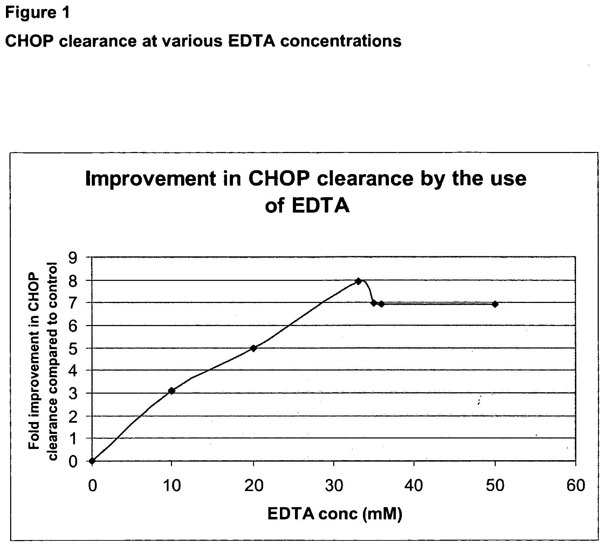

The results of repeated control experiments and EDTA-containing experiments at different levels of EDTA in the loading fluid are presented in table 1. This clearance is presented graphically in Figure 1. CHOP clearance increases linearly with EDTA concentration up to a peak around 35mM (with some allowance for experimental variation), with no further gains observed past this point. CHOP levels were quantified by Enzyme Linked Immunosorbent Assay (ELISA) (Cygnus Technologies catalogue #F015).

### Example 1d: Removal of aggregated or degraded forms of rIX-FP

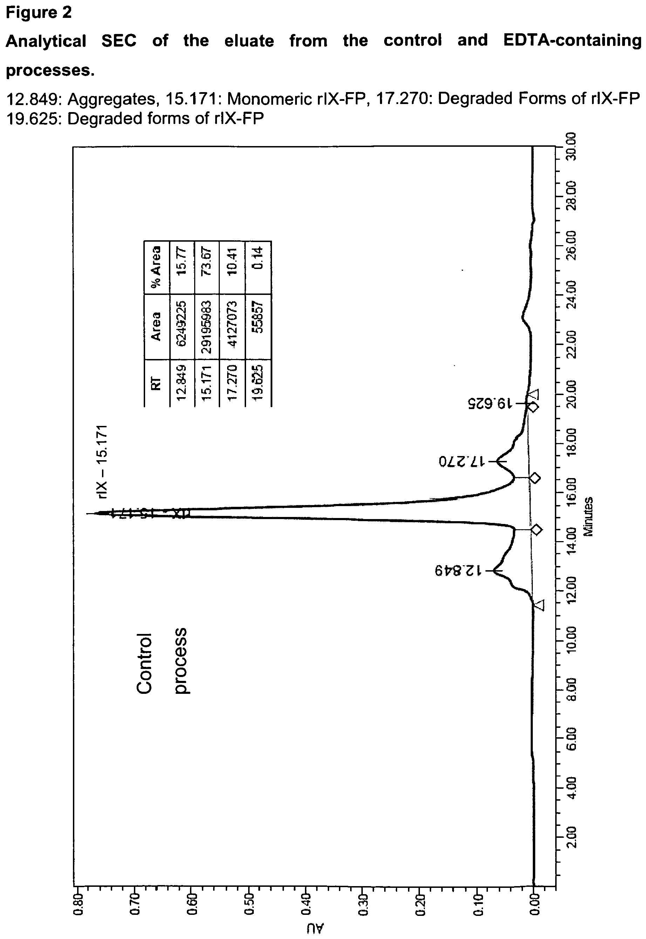

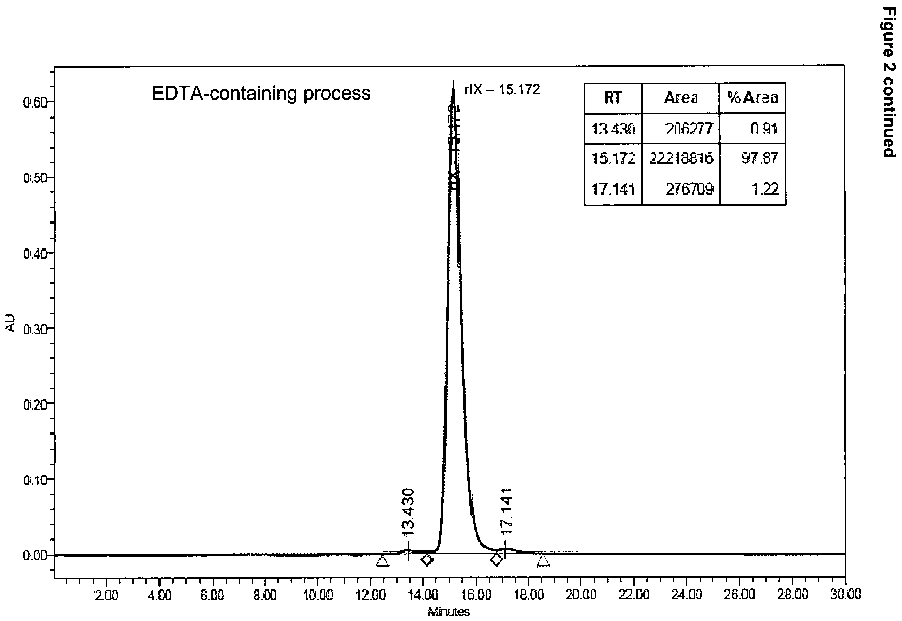

The major protein species observed via analytical size exclusion chromatography (SEC) on High Performance Liquid Chromatography are presented in Table 2. The use of the EDTA-containing process significantly reduces the level of both aggregates and fragments (breakdown products) in the elution. This can also be seen in Figure 2, which presents SEC traces of the eluted product from the control and EDTA-containing processes.

### Example 1e: Removal of Factor IXalpha

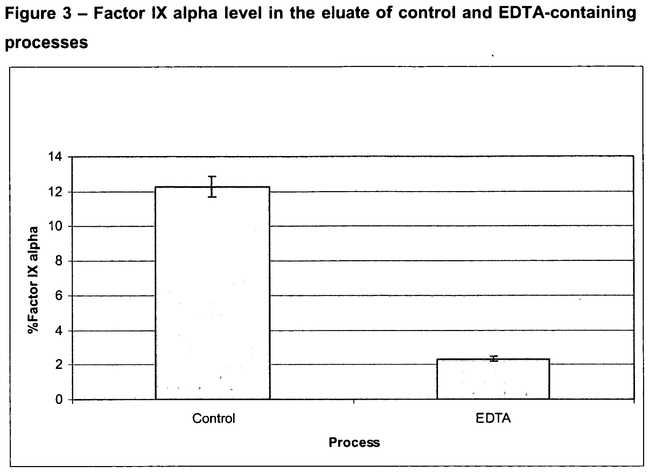

Factor IXalpha is one specific proteolytic breakdown product resulting from cleavage at only the Arg145 - Ala146 activation site. This form of protein is not separable by SEC, as it remains together via disulphide bonds and is thus a specific problem in the purification of FIX. The use of the EDTA-containing process improves clearance of this species as shown in Figure 3, measured by analytical capillary electrophoresis operated under reduced and denaturing conditions.

### Example 1f: Large scale purification of rIX-FP

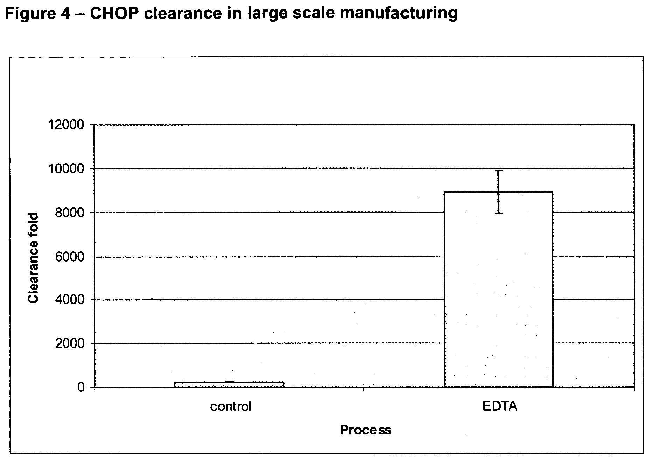

500L of clarified cell culture fluid was adjusted to a conductivity on ≤13.5 mS/cm by dilution with 20mM Tris-HCI, pH7.0. Disodium-EDTA was then added to this solution to a level of 35mM bringing the conductivity back up to a level comparable to that of the unadjusted feedstock (≤18 mS/cm). This material was then applied to a POROS® HQ 50 substantially as described in Example 1b. The host-cell clearance achieved at this scale is presented in Table 3 and Figure 4, comparing it to that achieved in large scale applications of the control (Example 1a) process.

**Table 1 - Experimental data from control (no added EDTA) and EDTA-containing experiments. EDTA was added into the clarified cell culture fluid at various concentrations. In all EDTA-containing experiments the equilibration and post-load wash buffers were the same (50mM MES, 100mM NaCl, 50mM EDTA, pH5.0).**

| **Experiment (EDTA concentration in loading fluid)** | **Step** | **Activity** | | | **CHOP** | | | |
|---|---|---|---|---|---|---|---|---|
| | | **Total IU** | **Recovery** | **IU/OD** | **Total (ug)** | **CHOP/IU** | **Clearance fold** | **X fold improvement in clearance over control process average** |
| Control 1 | Loading Fluid | 3075 | | | 28221 | 9.18 | | **0 (Average clearance = 132 fold)** |
| | Elution Fluid | 1987 | 64.6 | ND | 143 | 0.07 | 198 | |
| Control 2 | Loading Fluid | 3191 | | | 13904 | 4.36 | | |
| | Elution Fluid | 2406 | 93.8 | 53.7 | 130 | 0.054 | 107 | |
| Control 3 | Loading Fluid | 38397 | | | 195779 | 5.10 | | |
| | Elution Fluid | 25152 | 65.5 | ND | 2142 | 0.09 | 91 | |
| 10mM EDTA | Loading Fluid | 3212 | | | 13772 | 4.46 | | |
| | Elution Fluid | 2486 | 77.4 | 68.7 | 34 | 0.014 | 406 | **3** |
| 20mM EDTA | Loading Fluid | 3212 | | | 11554 | 3.75 | | |
| | Elution Fluid | 2695 | 83.9 | 68.7 | 17.65 | 0.0065 | 655 | **5** |
| 33mM EDTA | Loading Fluid | 3269 | | | 14556 | 4.45 | | |
| | Elution Fluid | 3117 | 95.4 | 89.5 | 14 | 0.004 | 1048 | **7.9** |
| 35mM EDTA | Loading Fluid | 3212 | | | 12545 | 3.75 | | |
| | Elution Fluid | 2819 | 87.8 | 80 | 13.6 | 0.0048 | 922 | **7.0** |
| 36mM EDTA | Loading Fluid | 2776 | | | 28221 | 10.17 | | |
| | Elution Fluid | 2605 | 89.03 | ND | 31 | 0.012 | 914 | **6.9** |
| 50mM EDTA | Loading Fluid | 3212 | | | 12697.4 | 3.046 | | |
| | Elution Fluid | 2561 | 79.7 | 84.5 | 13.94 | 0.005 | 911 | **6.9** |

**Table 2 - % abundance of each form of rIX-FP in the elution as determined by analytical SEC**

| **Experiment (EDTA concentration in Loading Fluid)** | **Aggregate** | **Monomer** | **Fragments** |
|---|---|---|---|
| Control 1 | 6.91 | 83.63 | 9.46 |
| Control 2 | 6.23 | 87.47 | 6.3 |
| Control 3 | 9.89 | 85.0 | 5.11 |
| 10mM EDTA | 3.84 | 92.2 | 3.95 |
| 20mM EDTA | 3.79 | 93.16 | 3.04 |
| 33mM EDTA | 3.66 | 93.6 | 2.75 |
| 35mM EDTA | 3.52 | 94.43 | 2.5 |
| 36mM EDTA | 1.75 | 92.46 | 5.79 |
| 50mM EDTA | 2.33 | 94.89 | 2.78 |

**Table 3 - Clearance of CHOP at large scale by the use of EDTA**

| Process type | Batch | CHOP amount (mg) | | CHOP clearance fold | X fold improvement in CHOP clearance compared to control process average |
|---|---|---|---|---|---|
| | | Loading Fluid | Elution Fluid | | |
| Control | 1 | 11205 | 34.6 | 324 | 0 (Average clearance = 252 fold) |
| | 2 | 10277 | 46.7 | 220 | |
| | 3 | 8896 | 37.8 | 235 | |
| | 4 | 12763 | 49.2 | 259 | |
| | 5 | 7748 | 34.7 | 223 | |
| EDTA-containing | 6 | 6349 | 0.9 | 7054 | 24 |
| | 7 | 14628 | 1.4 | 10449 | 41 |
| | 8 | 7996 | 0.84 | 9519 | 38 |

### Reference Example 2: Method for improved purification of rIX-FP on POROS® -HQ in the presence of chemical compounds different from EDTA in the load buffer

The effect of different chemical compounds different from EDTA was investigated using a high throughput batch screening approach with 96 well filter microplates. Investigated compounds were nitrilotriacetic acid (NTA), ethylenediamine-*N*,*N'-*disuccinic acid (EDDS), diethylene triamine pentaacetic acid (DTPA) and ethylenediamine tetra(methylene phosphonic acid) (EDTMP). Representative clarified harvest material was diluted 1:1 with 2x equilibration buffers and adjusted to a conductivity of 16.5 mS/cm with final compound concentrations of 10, 20, 40 and 60 mM or no added compound for the control runs. Different compound concentrations were screened because optimal compound concentrations might be different from EDTA and pH and conductivities were not optimized for other compounds. In table 4 the buffers or solutions for each step are listed.

**Table 4 - Buffers used for high throughput screening of different compounds different from EDTA on POROS® 50 HQ**

| **Step** | **Buffer** |
|---|---|
| Equilibration | 50 mM MES, 85 mM NaCl (control) or combinations of NaCl and chemical compounds (10, 20, 40 or 60 mM) for a total of 85 mM, pH 5.0, conductivity 17 mS/cm |
| Load | Clarified harvest diluted 1:1 with 2x equilibration buffers (100 mM MES, 170 mM NaCl (control) or combinations of NaCl and chemical compounds (20, 40, 80 or 120 mM) for a total of 170 mM) and adjusted to a conductivity of 16.5 mS/cm |
| Post-load wash | 50 mM MES, 85 mM NaCl (control) or combinations of NaCl and chemical compounds (10, 20, 40 or 60 mM) for a total of 85 mM, pH 5.0, conductivity 17 mS/cm |
| Wash 1 | 50 mM MES, 195 mM NaCl, 2 mM CaCI2, pH 5.0 |
| Wash 2 | 50 mM TRIS-HCl, 100 mM NaCl, pH 8.5 |
| Wash 3 | 50 mM TRIS-HCl, 100 mM NaCl, 10 mM CaCI2, pH 8.5 |
| Elution | 50 mM TRIS-HCl, 100 mM NaCl, 30 mM CaCI2, pH 8.5 |

For incubation steps filter plates were agitated on a microplate stirrer at 1100 rpm for the designated incubation time and liquid was removed by using a vacuum manifold device into a deep well storage plate. Every set of experiment was done in quadruplicates and average results were used.

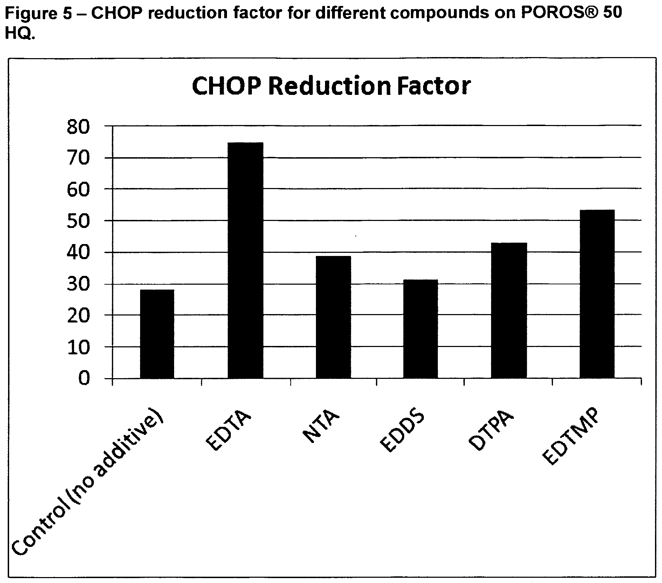

In detail POROS® 50 HQ resin slurries were transferred to 96 well filter plates (0.65 µm PVDF membrane) and the storage buffer was removed from the resins by vacuum. Resins were incubated with the respective equilibration buffers for 5 min. Adjusted load material was incubated with the resins for a total of 40 mins and flowthrough was collected. The resins were further washed with the respective equilibration buffers for 5 min and the post-load wash fractions were collected. All resins were incubated with wash 1, 2 and 3 buffers for 5 min each. The resins were eluted with 150 µL of elution buffer for 5 min. Load material, flowthrough, post-load wash and elution fractions were analyzed by Albumin Blue 580 assay (Sigma Aldrich catalogue #05497) for human albumin content. CHOP content was analyzed by Enzyme Linked Immunosorbent Assay (ELISA) (Cygnus Technologies catalogue #F015). Table 5 shows results for the CHOP Reduction Factor and the Improvement of the CHOP Reduction Factor of the EDTA process and other compounds vs. the control process using a high throughput screening approach and in Figure 5 results are presented as a chart.

**Table 5 - Improvement in Product content per CHOP and CHOP reduction for high throughput screening of different compounds different from EDTA on POROS® 50 HQ**

| **Additive** | **Concentration [mM]** | **Product per CHOP [mg/mg]** | | **CHOP Reduction Factor** | |
|---|---|---|---|---|---|
| | | **Load** | **Eluate** | **Factor** | **Compared to control [%]** |
| Control (no additive) | N/A | 5.5 (average) | 155 (average) | 28.2 (average) | --- |
| EDTA | 40 | 4.8 | 359 | 74.8 | 165 |
| NTA | 40 | 9.4 | 367 | 39.0 | 38 |
| EDDS | 40 | 9.4 | 294 | 31.3 | 11 |
| DTPA | 40 | 10.2 | 438 | 43.0 | 52 |
| EDTMP | 5 | 9.2 | 490 | 53.3 | 89 |

### Reference Example 3: Method for improved purification of rIX-FP in the presence of EDTA in the load buffer but using different anion exchange matrices

The effect of different anion exchange matrices different from POROS® 50 HQ was investigated using a high throughput batch screening approach with 96 well filter microplates. Investigated anion exchange matrices were Macro-Prep® 25Q (Bio-Rad), Macro-Prep® DEAE (Bio-Rad) and Cellufine Q-500 (Chisso). Representative clarified harvest material was diluted 1:1 with 2x equilibration buffers and adjusted to a conductivity of 16.5 mS/cm with final EDTA concentrations of 10, 20, 40 and 60 mM or no added compound for the control runs. Different EDTA concentrations were screened because optimal EDTA concentration might be different from POROS® 50 HQ and pH and conductivities were not optimized for other anion exchange matrices. In table 6 the buffers or solutions for each step are listed.

**Table 6 - Buffers used for high throughput screening of different anion exchange matrices using EDTA**

| **Step** | **Buffer** |
|---|---|
| Equilibration | 50 mM MES, 85 mM NaCl (control) or combinations of NaCl and EDTA (10, 20, 40 or 60 mM) for a total of 85 mM, pH 5.0, conductivity 17 mS/cm |
| Load | Clarified harvest diluted 1:1 with 2x equilibration buffers (100 mM MES, 170 mM NaCl (control) or combinations of NaCl and EDTA (20, 40, 80 or 120 mM) for a total of 170 mM) and adjusted to a conductivity of 16.5 mS/cm |
| Post-load wash | 50 mM MES, 85 mM NaCl (control) or combinations of NaCl and EDTA (10, 20, 40 or 60 mM) for a total of 85 mM, pH 5.0, conductivity 17 mS/cm |
| Wash 1 | 50 mM MES, 195 mM NaCl, 2 mM CaCI2, pH 5.0 |
| Wash 2 | 50 mM TRIS-HCl, 100 mM NaCl, pH 8.5 |
| Wash 3 | 50 mM TRIS-HCl, 100 mM NaCl, 10 mM CaCI2, pH 8.5 |
| Elution | 50 mM TRIS-HCl, 100 mM NaCl, 30 mM CaCI2, pH 8.5 |

For incubation steps filter plates were agitated on a microplate stirrer at 1100 rpm for the designated incubation time and liquid was removed by using a vacuum manifold device into a deep well storage plate. Every set of experiment was done in quadruplicates and average results were used.

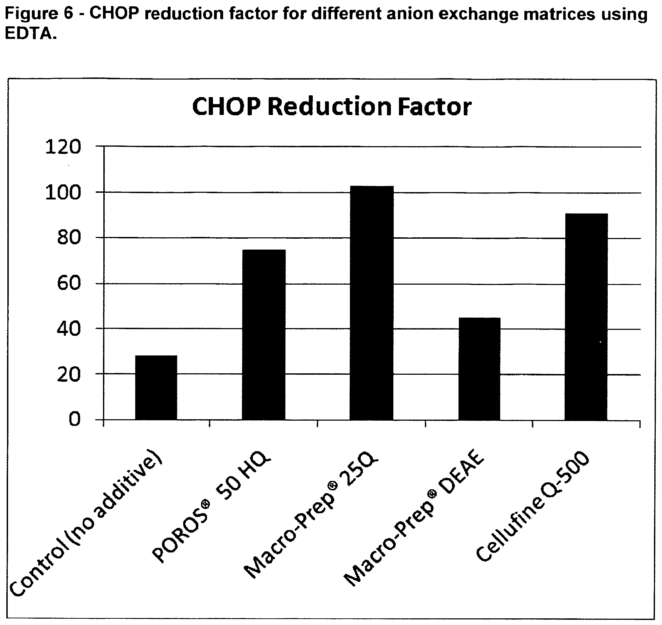

In detail anion exchange resin slurries were transferred to 96 well filter plates (0.65 µm PVDF membrane) and the storage buffer was removed from the resins by vacuum. Resins were incubated with the respective equilibration buffers for 5 min. Adjusted load material was incubated with the resins for a total of 40 mins and flowthrough was collected. The resins were further washed with the respective equilibration buffers for 5 min and the post-load wash fractions were collected. All resins were incubated with wash 1, 2 and 3 buffers for 5 min each. The resins were eluted with 150 µL of elution buffer for 5 min. Load material, flowthrough, post-load wash and elution fractions were analyzed by Albumin Blue 580 assay (Sigma Aldrich catalogue #05497) for human albumin content. CHOP content was analyzed by Enzyme Linked Immunosorbent Assay (ELISA) (Cygnus Technologies catalogue #F015). Table 7 shows results for the CHOP Reduction Factor and the Improvement of the CHOP Reduction Factor for the POROS® process and the other anion exchange matrices vs. the control using a high throughput screening approach and in Figure 6 results are presented as a chart.

**Table 7 - Improvement in Product content per CHOP and CHOP reduction for high throughput screening of different anion exchange matrices using EDTA**

| **Resin** | **EDTA Concentration [mM]** | **Product per CHOP [mg/mg]** | | **CHOP Reduction Factor** | |
|---|---|---|---|---|---|
| | | **Load** | **Eluate** | **Factor** | **Compared to control [%]** |
| Control (no additive) | N/A | 5.5 (average) | 155 (average) | 28.2 (average) | --- |
| POROS® 50 HQ | 40 | 4.8 | 359 | 74.8 | 165 |
| Macro-Prep® 25Q | 40 | 4.8 | 494 | 103.0 | 265 |
| Macro-Prep® DEAE | 40 | 6.3 | 283 | 45.0 | 60 |
| Cellufine Q-500 | 40 | 6.3 | 573 | 91.0 | 223 |

### Reference Example 4: Method for improved purification of rIX-FP on POROS® -HQ in the presence of EDTA in the wash buffer

The effect of having the EDTA solely in the post-load wash buffer on POROS® 50 HQ was investigated using a high throughput batch screening approach with 96 well filter microplates. Representative clarified harvest material was diluted 1:1 with 2x equilibration buffers and adjusted to a conductivity of 16.5 mS/cm with no added EDTA. An EDTA concentration of 40 mM in the post-load wash buffer was used. In table 8 the buffers or solutions for each step are listed.

**Table 8 - Buffers used for high throughput screening of different anion exchange matrices using EDTA**

| **Step** | **Buffer** |
|---|---|
| Equilibration | 50 mM MES, 85 mM NaCl |
| Load | Clarified harvest diluted 1:1 with 2x equilibration buffers (100 mM MES, 170 mM NaCl and adjusted to a conductivity of 16.5 mS/cm |
| Post-load wash | 50 mM MES, 85 mM NaCl (control) or 45 mM NaCl and 40 mM EDTA mM, pH 5.0, conductivity 17 mS/cm |
| Wash 1 | 50 mM MES, 195 mM NaCl, 2 mM CaCI2, pH 5.0 |
| Wash 2 | 50 mM TRIS-HCl, 100 mM NaCl, pH 8.5 |
| Wash 3 | 50 mM TRIS-HCl, 100 mM NaCl, 10 mM CaCI2, pH 8.5 |
| Elution | 50 mM TRIS-HCl, 100 mM NaCl, 30 mM CaCl2, pH 8.5 |

For incubation steps filter plates were agitated on a microplate stirrer at 1100 rpm for the designated incubation time and liquid was removed by using a vacuum manifold device into a deep well storage plate. Every set of experiment was done in quadruplicates and average results were used.

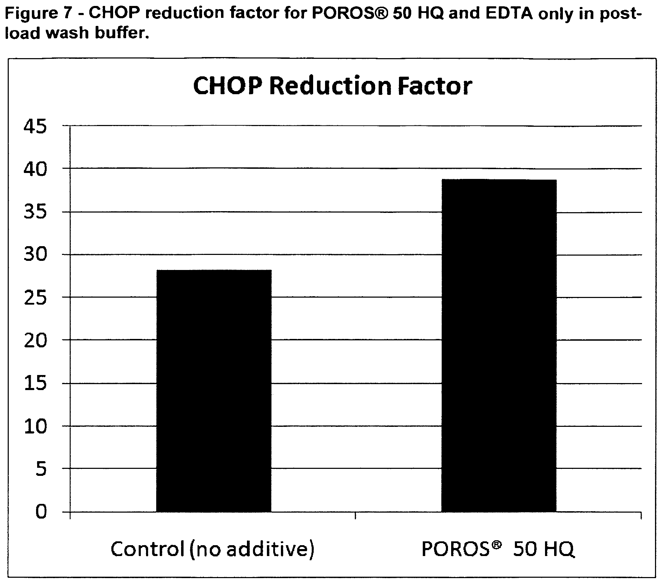

POROS® 50 HQ resin slurries were transferred to 96 well filter plates (0.65 µm PVDF membrane) and the storage buffer was removed from the resins by vacuum. Resins were incubated with the respective equilibration buffers for 5 min. Adjusted load material was incubated with the resins for a total of 40 mins and flowthrough was collected. The resins were further washed with the respective equilibration buffers for 5 min and the post-load wash fractions were collected. All resins were incubated with wash 1, 2 and 3 buffers for 5 min each. The resins were eluted with 150 µL of elution buffer for 5 min. Load material, flowthrough, post-load wash and elution fractions were analyzed by Albumin Blue 580 assay (Sigma Aldrich catalogue #05497) for human albumin content. CHOP content was analyzed by Enzyme Linked Immunosorbent Assay (ELISA) (Cygnus Technologies catalogue #F015). Table 9 shows results for the CHOP Reduction Factor and the Improvement of the CHOP Reduction Factor for EDTA only being in the post-load wash buffer vs. the control and in Figure 7 results are presented as a chart.

**Table 9 - Improvement in Product content per CHOP and CHOP reduction for high throughput screening of EDTA only in post-load wash buffer using POROS® 50 HQ**

| **Resin** | **EDTA Concentration [mM]** | **Product per CHOP [mg/mg]** | | **CHOP Reduction Factor** | |
|---|---|---|---|---|---|
| | | **Load** | **Eluate** | **Factor** | **Compared to control [%]** |
| Control (no additive) | N/A | 5.5 (average) | 155 (average) | 28.2 (average) | --- |
| POROS® 50 HQ | 40 (only in post-load wash buffer) | 7.4 | 287 | 38.8 | 38 |

### Reference Example 5: Method for improved purification of a monoclonal antibody on the AEX matrix Fractogel® EMD TMAE in the presence of EDTA in the load buffer

The effect of EDTA on the purification of a monoclonal antibody on an anion exchange matrix was investigated using a high throughput batch screening approach with 96 well filter microplates. Representative clarified harvest material was diluted 1:1 with 2x equilibration buffers with final EDTA concentrations of 5, 10, and 20 mM or no added compound for the control runs. Different EDTA concentrations were screened because purification conditions were not optimized. Table 10 lists the buffers used for each step in the screening.

**Table 10 - Buffers used for high throughput screening of a monoclonal antibody purification on Fractogel® EMD TMAE using EDTA**

| **Step** | **Buffer** |
|---|---|
| Equilibration | 10 mM TRIS, pH 9.0, 20 mM NaCl (control) or combinations of NaCl and EDTA to a final concentration of 20 mM with EDTA concentrations of 5, 10 or 20 mM |
| Load | Clarified harvest adjusted to pH 9.0 and 20 mM NaCl (control) or or combinations of NaCl and EDTA to a final concentration of 20 mM with EDTA concentrations of 5, 10 or 20 mM |
| Post-load wash | 10 mM TRIS, pH 9.0, 20 mM NaCl (control) or combinations of NaCl and EDTA to a final concentration of 20 mM with EDTA concentrations of 5, 10 or 20 mM |
| Wash | 10 mM TRIS, pH 9.0, 20 mM NaCl |
| Elution | 10 mM TRIS, pH 9.0, 1 M NaCl |

For incubation steps filter plates were agitated on a microplate stirrer at 1100 rpm for the designated incubation time and liquid was removed by using a vacuum manifold device into a deep well storage plate. Every set of experiment was done in quadruplicates and average results were used.

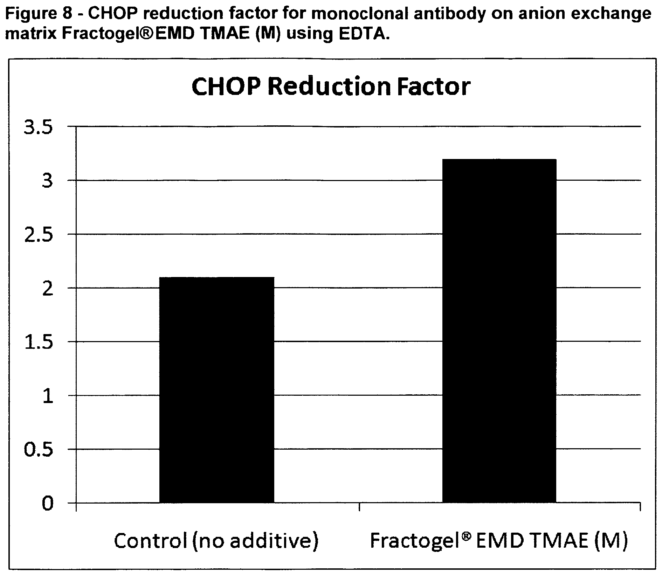

Fractogel® EMD TMAE resin slurries were transferred to 96 well filter plates (0.65 µm PVDF membrane) and the storage buffer was removed from the resins by vacuum. Resins were incubated with the respective equilibration buffers for 5 min. Adjusted load material was incubated with the resins for a total of 40 mins and flowthrough was collected. The resins were further washed with the respective equilibration buffers for 5 min and the post-load wash fractions were collected. All resins were washed with equilibration buffer without added compounds for 5 min each. The resins were eluted with 150 µL of elution buffer for 5 min. Load material, flowthrough, post-load wash and elution fractions were analyzed by Protein A HPLC for IgG content. CHOP content was analyzed by Enzyme Linked Immunosorbent Assay (ELISA) (Cygnus Technologies catalogue #F015). Table 11 shows results for the CHOP Reduction Factor and the Improvement of the CHOP Reduction Factor for increasing EDTA concentrations vs. control for the purification of a monoclonal antibody on Fractogel® EMD TMAE using a high throughput screening approach and in Figure 8 results are presented as a chart.

**Table 11 - Improvement in Product content per CHOP and CHOP reduction for high throughput screening of a monoclonal antibody purification on Fractogel® EMD TMAE using EDTA**

| **Resin** | **EDTA Concentration [mM]** | **Product per CHOP [mg/mg]** | | **CHOP Reduction Factor** | |
|---|---|---|---|---|---|
| | | **Load** | **Eluate** | **Factor** | **Compared to control [%]** |
| Control (no additive) | N/A | 14.8 | 31.5 | 2.1 | --- |
| Fractogel ® EMD TMAE (M) | 10 | 14.7 | 46.4 | 3.2 | 52 |

### Example 6: Method for improved purification of a monoclonal antibody on the CEX matrix Fractogel® EMD SO3 in the presence of DPA in the load buffer

The effect of 2,2'-dipicolylamine (DPA) on the purification of a monoclonal antibody on a cation exchange matrix was investigated using a high throughput batch screening approach with 96 well filter microplates. Representative clarified harvest material was diluted 1:1 with 2x equilibration buffers with final DPA concentrations of 5, 10, and 20 mM or no added compound for the control runs. Different DPA concentrations were screened because purification conditions were not optimized. Table 12 lists the buffers used for each step in the screening.

**Table 12 - Buffers used for high throughput screening of a monoclonal antibody purification on Fractogel® EMD SO3 using DPA**

| **Step** | **Buffer** |
|---|---|
| Equilibration | 10 mM MES, pH 6.0, 20 mM NaCl (control) or combinations of NaCl and EDTA to a final concentration of 20 mM with DPA concentrations of 5, 10 or 20 mM |
| Load | Clarified harvest adjusted to pH 6.0 and 20 mM NaCl (control) or combinations of NaCl and DPA to a final concentration of 20 mM with DPA concentrations of 5, 10 or 20 mM |
| Post-load wash | 10 mM MES, pH 6.0, 20 mM NaCl (control) or combinations of NaCl and EDTA to a final concentration of 20 mM with DPA concentrations of 5, 10 or 20 mM |
| Wash | 10 mM MES, pH 6.0, 20 mM NaCl |
| Elution | 10 mM MES, pH 6.0, 300 mM NaCl |

For incubation steps filter plates were agitated on a microplate stirrer at 1100 rpm for the designated incubation time and liquid was removed by using a vacuum manifold device into a deep well storage plate. Every set of experiment was done in quadruplicates and average results were used.

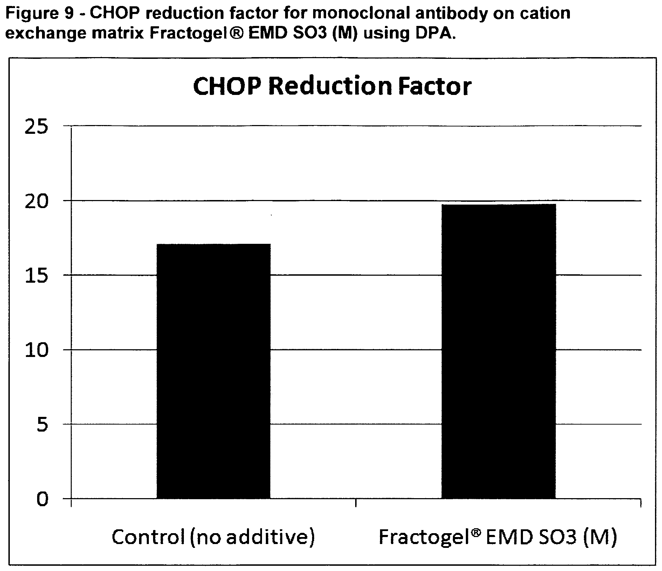

Fractogel® EMD SO3 resin slurries were transferred to 96 well filter plates (0.65 µm PVDF membrane) and the storage buffer was removed from the resins by vacuum. Resins were incubated with the respective equilibration buffers for 5 min. Adjusted load material was incubated with the resins for a total of 40 mins and flowthrough was collected. The resins were further washed with the respective equilibration buffers for 5 min and the post-load wash fractions were collected. All resins were washed with equilibration buffer without added compounds for 5 min each. The resins were eluted with 150 µL of elution buffer for 5 min. Load material, flowthrough, post-load wash and elution fractions were analyzed by Protein A HPLC for IgG content. CHOP content was analyzed by Enzyme Linked Immunosorbent Assay (ELISA) (Cygnus Technologies catalogue #F015). Table 13 shows results for the CHOP Reduction Factor and the Improvement of the CHOP Reduction Factor for increasing DPA concentrations vs. control for the purification of a monoclonal antibody on Fractogel® EMD SO3 using a high throughput screening approach and in Figure 9 results are presented as a chart.

**Table 13 - Improvement in Product per CHOP content and CHOP reduction for high throughput screening of a monoclonal antibody purification on Fractogel® EMD SO3 using DPA**

| **Resin** | **DPA Concentration [mM]** | **Product per CHOP [mg/mg]** | | **CHOP Reduction Factor** | |
|---|---|---|---|---|---|
| | | **Load** | **Eluate** | **Factor** | **Compared to control [%]** |
| Control (no additive) | N/A | 14.9 | 255 | 17.1 | --- |
| Fractogel ® EMD SO3 (M) | 20 | 14.8 | 293 | 19.8 | 16 |

### Reference Example 7: Method for improved purification of rVlla-FP on the AEX matrix POROS® 50 HQ using EDTA as additive

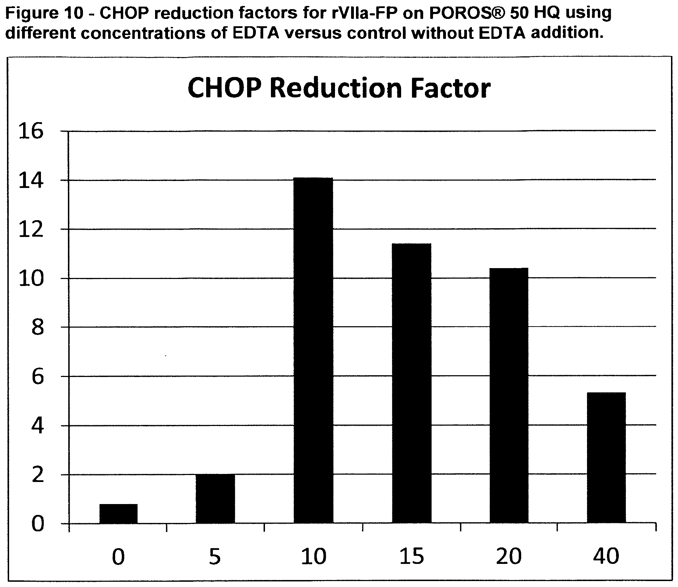

Recombinant albumin fused FVIIa was expressed in CHO-S cells as described by Weimer et al. (Thromb. Hemost. (2008) Vol 99 (4) pp. 659-67. Clarified cell culture fluid was adjusted to different EDTA, pH and conductivity levels and applied to an anion exchange column packed with POROS® HQ 50 (Applied Biosystems). This column had previously been equilibrated with 20mM HEPES, 50mM NaCl, pH6.2, adjusted to the same conditions as the clarified cell culture fluid, e.g. EDTA spike. The load was followed by a post-load wash with the respective equilibration buffer. Any unbound material was washed from the column with 20mM HEPES, 100mM NaCl, pH6.2. The purified rVlla-FP was then eluted from the column with 20mM HEPES, 150mM NaCl, 10mM CaCl₂, pH6.2. rFVIIa-FP activity was measured with a chromogenic activity assay and CHOP content was analyzed by Enzyme Linked Immunosorbent Assay (ELISA) (Cygnus Technologies catalogue #F015). Table 14 shows results for the CHOP Reduction Factor and the Improvement of the CHOP Reduction Factor for the POROS® process and the vs. the control for different EDTA concentrations and in Figure 10 the results are presented as a chart.

**Table 14 - Improvement in FVIIa content per CHOP and CHOP reduction for EDTA on POROS® 50 HQ**

| **EDTA Concentration (mM)** | **Product per CHOP (mg/mg)** | | **CHOP Reduction Factor** | |
|---|---|---|---|---|
| | **Load** | **Eluate** | **Factor** | **Compared to Control (%)** |
| **0** | 55 | 43 | 0.8 | --- |
| **5** | 299 | 586 | 2.0 | 150 |
| **10** | 216 | 3042 | 14.1 | 1663 |
| **15** | 452 | 5159 | 11.4 | 1325 |
| **20** | 361 | 3769 | 10.4 | 1200 |
| **40** | 487 | 2564 | 5.3 | 563 |

## Claims

1. Process for the purification of a polypeptide of interest by cation-exchange chromatography wherein a chemical compound is added in a concentration of at least 7 mM
a) to an equilibration fluid of the cation-exchange matrix wherein the equilibration fluid is adjusted such that at least part of the chemical compound in the equilibration fluid binds to the cation-exchange matrix due to a charge that is opposite to the charge of the cation-exchange matrix
and/or
b) to a loading fluid which is applied to the cation-exchange matrix and which comprises the polypeptide of interest wherein the loading fluid is adjusted such that at least part of the chemical compound and at least part of the polypeptide of interest in the loading fluid bind to the cation-exchange matrix due to a charge that is opposite to the charge of the cation-exchange matrix
and/or
c) to a washing fluid which is used to wash the cation-exchange matrix once the polypeptide of interest has bound to the cation-exchange matrix due to a charge that is opposite to the charge of the cation-exchange matrix wherein the washing fluid is adjusted such that at least part of the chemical compound in the washing fluid binds to the cation-exchange matrix due to a charge that is opposite to the charge of the cation-exchange matrix and that at least part of the polypeptide of interest and at least part of the already bound chemical compound if added at step a) or b) continue to bind to the cation-exchange matrix due to a charge that is opposite to the charge of the cation-exchange matrix
thereby increasing the amount of the polypeptide of interest bound to the cation-exchange matrix relative to the amount of one or more impurities bound to the cation-exchange matrix before the cation-exchange matrix is eluted and thereby leading to an increased ratio of the polypeptide of interest to one or more impurities in the eluate as compared to the same process wherein the chemical compound is added at a concentration of below 7mM,
wherein the chemical compound is selected from a list consisting of chemical structures with amino groups and/or cationic amino acid polymers, such as tetraethylene pentamine (TEPA), dipicolylamine (DPA), poly-lysine, poly-arginine and poly-histidine.

2. Process according to claim 1 wherein the impurities comprise host cell proteins and/or host cell nucleic acids and/or product-related contaminants and/or viruses and/or prions and/or endotoxins and/or process-related contaminants.

## Patentansprüche

1. Verfahren zur Reinigung eines Polypeptids von Interesse durch Kationenaustauschchromatographie, wobei eine chemische Verbindung in einer Konzentration von mindestens 7 mM gegeben wird
a) zu einer Äquilibrierungsflüssigkeit der Kationenaustauschmatrix, wobei die Äquilibrierungsflüssigkeit so eingestellt wird, dass mindestens ein Teil der chemischen Verbindung in der Äquilibrierungsflüssigkeit aufgrund einer zur Ladung der Kationenaustauschmatrix entgegengesetzten Ladung an die Kationenaustauschmatrix bindet
und/oder
b) zu einer Ladeflüssigkeit, die auf die Kationenaustauschmatrix aufgebracht wird und die das Polypeptid von Interesse umfasst, wobei die Ladeflüssigkeit so eingestellt ist, dass mindestens ein Teil der chemischen Verbindung und mindestens ein Teil des Polypeptids von Interesse in der Ladeflüssigkeit aufgrund einer zur Ladung der Kationenaustauschmatrix entgegengesetzten Ladung an die Kationenaustauschmatrix binden
und/oder
c) zu einer Waschflüssigkeit, die zum Waschen der Kationenaustauschmatrix verwendet wird, sobald das Polypeptid von Interesse aufgrund einer zur Ladung der Kationenaustauschmatrix entgegengesetzten Ladung an die Kationenaustauschmatrix gebunden hat, wobei die Waschflüssigkeit so eingestellt ist, dass mindestens ein Teil der chemischen Verbindung in der Waschflüssigkeit aufgrund einer zur Ladung der Kationenaustauschmatrix entgegengesetzten Ladung an die Kationenaustauschmatrix bindet und dass mindestens ein Teil des Polypeptids von Interesse und mindestens ein Teil der bereits gebundenen chemischen Verbindung, wenn sie in Schritt a) oder b) zugesetzt wurden, aufgrund einer zur Ladung der Kationenaustauschmatrix entgegengesetzten Ladung weiterhin an die Kationenaustauschmatrix binden,
wodurch die Menge des an die Kationenaustauschmatrix gebundenen Polypeptids von Interesse relativ zu der Menge einer oder mehrerer an die Kationenaustauschmatrix gebundener Verunreinigungen erhöht wird, bevor die lonenaustauschmatrix eluiert wird, und wodurch es zu einem erhöhten Verhältnis des Polypeptids von Interesse zu einer oder mehreren Verunreinigungen im Eluat im Vergleich zu demselben Verfahren, bei dem die chemische Verbindung in einer Konzentration von weniger als 7 mM zugesetzt wird, kommt, wobei die chemische Verbindung ausgewählt ist aus einer Liste, bestehend aus chemischen Strukturen mit Aminogruppen und/oder kationischen Aminosäurepolymeren umfasst, wie Tetraethylenpentamin (TEPA), Dipicolylamin (DPA), Polylysin, Polyarginin und Polyhistidin.

2. Verfahren nach Anspruch 1, wobei die Verunreinigungen Wirtszellproteine und/oder Wirtszellnukleinsäuren und/oder produktbedingte Kontaminanten und/oder Viren und/oder Prionen und/oder Endotoxine und/oder prozessbedingte Kontaminanten umfassen.

## Revendications

1. Processus destiné à la purification d'un polypeptide d'intérêt, par chromatographie échangeuse de cations, dans lequel un composé chimique est ajouté, en une concentration d'au moins 7 mM,
a) à un liquide d'équilibrage de la matrice échangeuse de cations, le liquide d'équilibrage étant ajusté tel qu'au moins une partie du composé chimique dans le liquide d'équilibrage se lie à la matrice échangeuse de cations en raison d'une charge qui est opposée à la charge de la matrice échangeuse de cations
et/ou
b) à un liquide de charge qui est appliqué à la matrice échangeuse de cations et qui comprend le polypeptide d'intérêt, le liquide de charge étant ajusté tel qu'au moins une partie du composé chimique et au moins une partie du polypeptide d'intérêt dans le liquide de charge se lient à la matrice échangeuse de cations en raison d'une charge qui est opposée à la charge de la matrice échangeuse de cations
et/ou
c) à un liquide de lavage qui est utilisé pour laver la matrice échangeuse de cations une fois que le polypeptide d'intérêt s'est lié à la matrice échangeuse de cations, en raison d'une charge qui est opposée à la charge de la matrice échangeuse de cations, le liquide de lavage étant ajusté tel qu'au moins une partie du composé chimique dans le liquide de lavage se lie à la matrice échangeuse de cations, en raison d'une charge qui est opposée à la charge de la matrice échangeuse de cations, et qu'au moins une partie du polypeptide d'intérêt et qu'au moins une partie du composé chimique déjà lié, s'il a été ajouté à l'étape a) ou à la b), continuent à se lier à la matrice échangeuse de cations en raison d'une charge qui est opposée à la charge de la matrice échangeuse de cations
en augmentant de ce fait la quantité du polypeptide d'intérêt liée à la matrice échangeuse de cations, par rapport à la quantité d'une ou de plusieurs impureté(s) liée à la matrice échangeuse de cations avant que la matrice échangeuse de cations soit éluée, et en conduisant de ce fait à un rapport accru du polypeptide d'intérêt sur une ou plusieurs impureté(s) dans la solution d'élution, en comparaison avec le même processus dans lequel le composé chimique est ajouté en une concentration inférieure à 7 mM, dans lequel le composé chimique est choisi à partir d'une liste constituée par des structures chimiques avec des groupes amino et/ou des polymères d'acides aminés cationiques, telles que la tétraéthylène pentamine (TEPA), la dipicolylamine (DPA), la poly-lysine, la poly-arginine et la poly-histidine.

2. Processus selon la revendication 1, dans lequel les impuretés comprennent des protéines de cellule hôte et/ou des acides nucléiques de cellule hôte et/ou des contaminants connexes au produit et/ou des virus et/ou des prions et/ou des endotoxines et/ou des contaminants connexes au processus.
